(19) Europäisches Patentamt
European Patent Office
Office européen des brevets

(11) **EP 3 979 911 B1**

(12) **FASCICULE DE BREVET EUROPEEN**

(45) Date de publication et mention
de la délivrance du brevet:
**26.07.2023   Bulletin 2023/30**

(21) Numéro de dépôt: **20732155.5**

(22) Date de dépôt: **04.06.2020**

(51) Classification Internationale des Brevets (IPC):
*A61B 5/1455* (2006.01)      *A61B 5/1491* (2006.01)

(52) Classification Coopérative des Brevets (CPC):
**A61B 5/14551; A61B 5/14552;** A61B 5/14542;
A61B 5/1491; A61B 5/6824

(86) Numéro de dépôt international:
**PCT/EP2020/065523**

(87) Numéro de publication internationale:
**WO 2020/249466 (17.12.2020 Gazette 2020/51)**

(54) **DISPOSITIF PORTABLE D'ESTIMATION DE LA PRESSION PARTIELLE DE GAZ SANGUIN**

TRAGBARE VORRICHTUNG ZUR BESTIMMUNG DES PARTIALDRUCKS VON BLUTGAS

PORTABLE DEVICE FOR ESTIMATING THE PARTIAL PRESSURE OF BLOOD GAS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **09.06.2019   FR 1906142**

(43) Date de publication de la demande:
**13.04.2022   Bulletin 2022/15**

(73) Titulaire: **COMMISSARIAT À L'ÉNERGIE
ATOMIQUE ET AUX
ÉNERGIES ALTERNATIVES
75015 Paris (FR)**

(72) Inventeurs:
• **GRANGEAT, Pierre
38054 Grenoble cedex 09 (FR)**
• **ACCENSI, Marc
38054 Grenoble cedex 09 (FR)**
• **GHARBI, Sadok
38054 Grenoble cedex 09 (FR)**
• **GRATEAU, Henri
38054 Grenoble cedex 09 (FR)**

(74) Mandataire: **INNOV-GROUP
310, avenue Berthelot
69372 Lyon Cedex 08 (FR)**

(56) Documents cités:
FR-A1- 2 408 353      US-A- 3 987 303
US-A1- 2001 034 479    US-A1- 2010 130 842
US-A1- 2013 281 806    US-A1- 2016 151 009

**Description**

**DOMAINE TECHNIQUE**

Le domaine technique de l'invention est la mesure d'un gaz dégagé par la peau d'un être vivant à l'aide d'un dispositif compact, de façon à estimer des paramètres physiologiques, en particulier la teneur de dioxyde de carbone dissous dans le sang. Le gaz peut notamment être du dioxyde de carbone.

**ART ANTERIEUR**

Certaines maladies respiratoires affectent les échanges gazeux entre le sang et l'air expiré. Ces derniers peuvent également être modifiés en cours de réanimation. Le sang contient des gaz dissous, dont l'oxygène et le dioxyde de carbone, dont les pressions partielles respectives reflètent les échanges gazeux se produisant au niveau des poumons, ces derniers étant usuellement désignés par le terme "hématose". Chez un sujet normal, la pression partielle de $CO_2$ est généralement comprise entre 35 et 45 mm Hg, soit entre 4.8 et 5.8 kPa.

Pour évaluer la quantité de $CO_2$ dissous dans le sang, on peut avoir recours à un prélèvement sanguin. Il s'agit d'une méthode invasive, pouvant être douloureuse et délicate à appliquer, en particulier en néonatologie. De plus, elle ne peut être appliquée que de façon ponctuelle. En dépit de ces inconvénients, sa fiabilité est validée par le corps médical et elle constitue une méthode de référence.

Une autre méthode consiste à estimer la teneur du sang en $CO_2$ de façon non invasive, en effectuant une mesure de la pression partielle de $CO_2$ diffusant à travers les tissus, et notamment la peau. Ce type d'analyse transcutanée a été introduit dans les années 1980. L'analyse transcutanée permet d'effectuer un suivi continu, par exemple pour suivre les effets immédiats d'une prise en charge thérapeutique. Elle peut également permettre de déterminer les instants auxquels une quantification précise, par prélèvement sanguin, est nécessaire. On comprend ainsi que les méthodes invasives et non invasives peuvent être combinées : l'une est précise est ponctuelle, tandis que l'autre peut être mise en oeuvre en oeuvre de façon continue.

Des dispositifs compacts permettant la mesure du $CO_2$ transcutané ont déjà été décrits. Citons par exemple la publication Eletr S "Cutaneous monitoring of systemic PCO2 on patients in the respiratory intensive care unit being weaned from the ventilator", acta Anaesthesiol Scand, vol. 22, n° 68, pp 123-127, 1978.

Le document US-2016/151009-A1 montre un dispositif de mesure d'un gaz transcutané émis à travers la peau, avec une chambre de collecte s'étendant entre une face de contact et une chambre de mesure, une source de chaleur, et des ouvertures formant une admission d'air. Les documents US-2013/281806-A1, US-2010/130842-A1, US-3987303-A, US-2001/034479-A1 et FR-2408353-A1 montrent d'autres dispositifs de mesure de gaz transcutané.

L'invention exposée ci-dessous est une amélioration des dispositifs existants. Elle met en oeuvre un dispositif de mesure non invasif, porté par un utilisateur, pour estimer une concentration d'un gaz d'intérêt émis de façon transcutanée, ce gaz d'intérêt pouvant par exemple être le dioxyde de carbone. La circulation du gaz dans le dispositif permet une collecte optimisée du gaz d'intérêt. Par ailleurs, le dispositif peut utiliser un capteur optique de gaz d'intérêt, dont les signaux mesurés sont traités de façon optimale.

**EXPOSE DE L'INVENTION**

Un premier objet de l'invention est un dispositif de mesure, destiné à être disposé sur la peau d'un utilisateur, l'utilisateur étant un être humain ou un animal, le dispositif s'étendant entre une face de contact, destinée à être appliquée contre la peau de l'utilisateur et une extrémité distale, le dispositif comportant une paroi latérale, s'étendant entre face de contact et l'extrémité distale, le dispositif comportant :

- au niveau de la face de contact, au moins une ouverture, configurée pour collecter un gaz d'intérêt transcutané, émis à travers la peau, l'ouverture étant pratiquée à travers la face de contact;
- une chambre de collecte, s'étendant entre la face de contact et une chambre de mesure;
- un capteur de gaz, disposé dans la chambre de mesure, et configuré pour mesurer une concentration de gaz d'intérêt s'écoulant dans la chambre de mesure ;
- une unité de traitement et de calcul, configurée pour estimer une teneur en gaz d'intérêt dans le corps de l'utilisateur à partir de la mesure issue du capteur de gaz;
- une source de chaleur, configurée pour porter la face de contact à une température supérieure à 37°C ;

le dispositif étant caractérisé en ce qu'il comporte :

- une ouverture, formant une admission d'air, ménagée à travers la paroi latérale, configurée pour admettre de l'air ambiant dans la chambre de collecte;

de telle sorte que sous l'effet de la température produite par la source de chaleur, un courant de convection d'air s'établit, l'air admis dans la chambre de collecte à travers la paroi latérale entraînant le gaz d'intérêt transcutané vers la chambre de mesure, l'air et le gaz d'intérêt transcutané formant un mélange gazeux s'étendant dans la chambre de mesure.

Le gaz d'intérêt peut notamment être du dioxyde de carbone.

Le dispositif peut comporter un capteur d'humidité pour déterminer un niveau d'humidité dans la chambre de mesure, l'unité de traitement et de calcul étant configurée pour prendre en compte le niveau d'humidité dans la chambre de mesure pour estimer une concentration de gaz d'intérêt dans la chambre de mesure.

Le dispositif peut comporter un capteur de pression, pour déterminer une pression dans la chambre de mesure, l'unité de traitement et de calcul étant configurée pour prendre en compte la pression dans la chambre de mesure pour estimer une pression partielle de gaz d'intérêt dans la chambre de collecte.

De préférence, le capteur de gaz est un capteur optique, comportant une source de rayonnement infra-rouge et un photodétecteur, la source de rayonnement infra-rouge et le photodétecteur étant agencés de telle sorte que le mélange gazeux s'écoule, dans la chambre de mesure, entre la source de rayonnement infra-rouge et le photodétecteur, le photodétecteur comportant deux voies de mesures configurées pour mesurer respectivement un rayonnement infra-rouge, émis par la source de rayonnement infra-rouge, et s'étant propagé à travers la chambre de mesure dans :

- une bande spectrale de mesure, correspondant à une bande spectrale d'absorption du gaz d'intérêt ;
- une bande spectrale de référence, considérée comme non absorbée par le gaz d'intérêt.

Le dispositif peut comporter :

- une chambre de mesure auxiliaire, configurée pour être occupée par l'air ambiant s'étendant autour du dispositif;
- un capteur auxiliaire de gaz d'intérêt, disposé dans la chambre de mesure auxiliaire, et configuré pour mesurer une concentration de gaz d'intérêt dans l'air ambiant occupant la chambre de mesure auxiliaire ;
  l'unité de traitement et de calcul étant configurée pour prendre en compte la mesure du capteur auxiliaire pour estimer la teneur en gaz d'intérêt dans le corps de l'utilisateur.

De préférence, le capteur auxiliaire de gaz d'intérêt est un capteur optique, comportant une source auxiliaire de rayonnement infra-rouge et un photodétecteur auxiliaire, la source auxiliaire de rayonnement infra-rouge et le photodétecteur auxiliaire étant agencés de telle sorte que l'air ambiant s'étend, dans la chambre de mesure auxiliaire, entre la source auxiliaire de rayonnement infra-rouge et le photodétecteur auxiliaire, le photodétecteur auxiliaire comportant deux voies de mesures configurées pour mesurer respectivement un rayonnement infra-rouge, émis par la source auxiliaire de rayonnement infra-rouge, et s'étant propagé à travers la chambre de mesure auxiliaire dans :

- une bande spectrale de mesure, correspondant à une bande spectrale d'absorption du gaz d'intérêt ;
- une bande spectrale de référence ($\lambda_2$), considérée comme non absorbée par le gaz d'intérêt.

Le dispositif peut comporter :

- un capteur de pression de l'air ambiant, l'air ambiant s'étendant autour du dispositif;
- et/ou un capteur de température de l'air ambiant ;
- et/ou un capteur de pression dans la chambre de collecte ;
- et/ou un capteur de température dans la chambre de collecte ;
- et/ou un capteur de température, configuré pour mesurer une température de la peau ;
- et/ou un capteur de température dans la chambre de mesure ;
- et/ou un capteur d'humidité, disposé dans la chambre de mesure auxiliaire ;
- et/ou un capteur de pression, dans la chambre de mesure auxiliaire ;
- et/ou un capteur de température, dans la chambre de mesure auxiliaire.

Le dispositif peut également comporter :

- un capteur d'humidité configuré pour mesurer l'humidité de l'air ambiant s'étendant autour du dispositif ;
- un capteur de température configuré pour mesurer la température de l'air ambiant s'étendant autour du dispositif ;
- un capteur de pression configuré pour mesurer la pression de l'air ambiant s'étendant autour du dispositif.

Un deuxième objet de l'invention est un procédé d'estimation d'une teneur en gaz d'intérêt dans le sang d'un utilisateur, à l'aide d'un dispositif selon le premier objet de l'invention, le dispositif étant appliqué de telle sorte que la face de contact est disposée en regard de la peau de l'utilisateur, le procédé comportant un échauffement de la face de contact ainsi qu'une admission d'air ambiant à travers la paroi latérale du dispositif, le procédé comportant :

a) une estimation d'une concentration de gaz d'intérêt dans la chambre de mesure;
b) à partir de la concentration de gaz d'intérêt résultant de l'étape a), calcul d'une pression partielle de gaz d'intérêt dans la chambre de collecte;
c) à partir de la pression partielle de gaz d'intérêt dans la chambre de collecte, résultant de l'étape b), estimation d'une pression partielle de gaz d'intérêt dissout dans le sang de l'utilisateur.

L'étape c) peut comporter les sous-étapes :

(i) à partir de la pression partielle de gaz d'intérêt dans la chambre de collecte, résultant de l'étape b), estimation d'une pression partielle de gaz d'intérêt transcutané, ayant diffusé à travers la peau de l'utilisateur;
(ii) à partir de la pression partielle de gaz d'intérêt transcutané résultant de la sous-étape (i), estimation d'une pression partielle de gaz d'intérêt dissous dans le sang de l'utilisateur.

Le procédé peut comporter une mesure d'une concentration de gaz d'intérêt dans l'air ambiant, la concentration ainsi mesurée étant prise en compte dans l'étape c).

Le procédé peut comporter une mesure d'une pression de l'air ambiant, la pression de l'air ambiant étant prise en compte dans l'étape c).

Selon un mode de réalisation, l'étape a) est mise en oeuvre à l'aide d'un capteur de gaz, disposé dans la chambre de mesure, et formé par une source de rayonnement infra-rouge et par un photodétecteur comportant deux voies de mesures configurées pour mesurer respectivement un rayonnement infra-rouge, émis par la source de rayonnement infra-rouge et s'étant propagé à travers la chambre de mesure dans :

- une bande spectrale de mesure, correspondant à une bande spectrale d'absorption du gaz d'intérêt ;
- une bande spectrale de référence considérée comme non absorbée par le gaz d'intérêt.

L'étape a) peut alors comporter :

- une comparaison de signaux respectivement générés par les deux voies de mesure ;
- une résolution d'un polynôme du second degré, mettant en oeuvre la comparaison ainsi que des paramètres prédéterminés, de façon à estimer la concentration de gaz d'intérêt dans la chambre de mesure.

La racine du polynôme peut être une puissance non entière de la concentration de gaz d'intérêt dans la chambre de mesure. Les paramètres du polynôme du second degré peuvent être définis lors d'une phase de calibration supervisée, à l'aide d'échantillons de calibration présentant une concentration connue en gaz d'intérêt.

L'étape a) peut comporter une mesure d'une teneur en vapeur d'eau dans la chambre de mesure, la teneur en vapeur d'eau étant prise en compte dans l'estimation de la concentration de gaz d'intérêt dans la chambre de mesure.

Le procédé peut comporter une mesure de la température dans la chambre de mesure et dans la chambre de collecte, l'étape b) comportant une prise en compte des températures ainsi mesurées.

Le procédé peut comporter une mesure de la pression dans la chambre de mesure et dans la chambre de collecte, l'étape b) comportant une prise en compte des pressions ainsi mesurées.

Le gaz d'intérêt peut être du dioxyde de carbone.

La bande spectrale de mesure peut être variable, de façon à adresser différents gaz d'intérêt.

Un troisième objet de l'invention est un procédé d'estimation d'une concentration d'un gaz d'intérêt dans un gaz, le gaz d'intérêt présentant une bande spectrale d'absorption, le gaz occupant une chambre de mesure, la chambre de mesure comportant une source de rayonnement infra-rouge et un photodétecteur, le photodétecteur comportant :

- une première voie, destinée à mesurer un rayonnement infra-rouge s'étant propagé à travers le gaz, et à générer un premier signal représentatif d'une quantité de lumière détectée dans la bande spectrale d'absorption ;
- une deuxième voie, destinée à mesurer un rayonnement infra-rouge s'étant propagé à travers le gaz, et à générer un deuxième signal représentatif d'une quantité de lumière détectée dans une bande spectrale de référence, dans laquelle l'absorption du rayonnement infra-rouge par le gaz d'intérêt est considérée comme négligeable ;

le procédé étant caractérisé en ce qu'il comporte :

- une comparaison des signaux respectivement générés par les deux voies de mesure ;
- une résolution d'un polynôme du second degré, mettant en oeuvre la comparaison, ainsi que des paramètres prédéfinis, de façon à estimer la concentration de gaz d'intérêt dans la chambre de mesure.

Une des racines du polynôme est alors une puissance, notamment une puissance non entière, de la concentration de l'espèce gazeuse analysée. Le calcul de la concentration peut nécessiter de calculer la racine du polynôme du second degré défini comme la puissance à un exposant égal à l'inverse de cette puissance non-entière.

Les paramètres du polynôme du second degré sont de préférence définis lors d'une phase de calibration supervisée, à l'aide d'échantillons de calibration présentant une concentration connue du gaz d'intérêt analysé.

Le procédé peut comporter une mesure d'une teneur en vapeur d'eau dans la chambre de mesure, la teneur en vapeur d'eau étant prise en compte dans l'estimation de la concentration de gaz d'intérêt analysé.

Le gaz d'intérêt peut notamment être du dioxyde de carbone. Le gaz peut comporter de l'air.

**FIGURES**

Les figures 1A et 1B sont des vues d'ensemble du dispositif.

Les figures 2A et 2B représentent les principaux éléments du dispositif.

Les figures 3A et 3B schématisent respectivement l'emplacement de la chambre de mesure ainsi que la chambre de mesure.

La figure 3C montre un détail de l'extrémité distale du dispositif.

La figure 4 illustre les flux de gaz dans le dispositif.

La figure 5 représente les principales étapes d'un procédé mettant en oeuvre le dispositif.

Les figures 6A et 6B représentent respectivement une chambre de mesure et une chambre de mesure auxiliaire.

Les figures 7A et 7B montrent des résultats expérimentaux obtenus.

**EXPOSE DE MODES DE REALISATION PARTICULIERS**

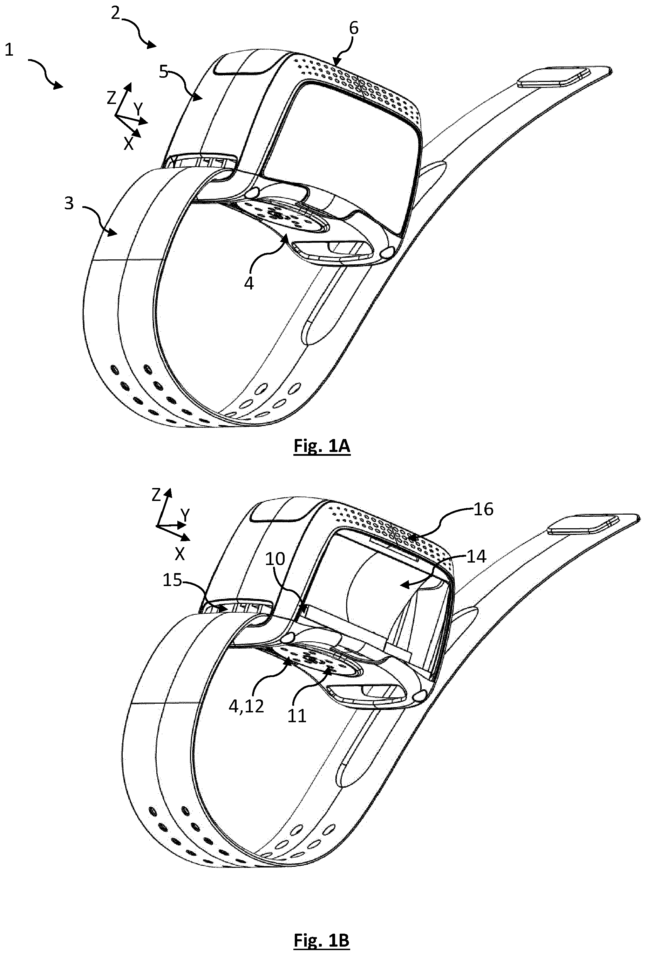

Les figures 1A et 1B sont des vues générales d'un exemple de dispositif 1 selon l'invention. Le dispositif 1 est destiné à être disposé au contact de la peau d'un utilisateur, humain ou animal, ou à une faible distance de la peau, typiquement inférieure à 1 cm. Il comporte un corps principal 2 ainsi qu'un élément de fixation 3, ce dernier étant, dans cet exemple, un bracelet. Le corps principal 2 comporte une face de contact 4, destinée à être apposée sur la peau. La face de contact est sensiblement planaire, au sens où elle s'étend parallèlement à un plan XY, certaines portions pouvant être inclinées par rapport au plan XY. Le corps principal comporte également une extrémité distale 6, opposée à la face de contact 4, et sensiblement parallèle à cette dernière. La surface de contact 4 et l'extrémité distale 6 sont reliées l'une à l'autre par une face latérale 5, s'étendant autour d'un axe transversal Z, perpendiculaire au plan XY.

Comme représenté sur la figure 1B, la face de contact 4 délimite une chambre de collecte 10. Cette dernière est, dans cet exemple, délimitée par une plaque, formant une partie de la face de contact 4, et dans laquelle sont ménagées des ouvertures transversales 11. Le dispositif comporte également un élément de chauffage 12, permettant de porter la face de contact 4, délimitant la chambre de collecte 10, à une température supérieure à 37°C, et de préférence comprise entre 40°C et 50°C, et de préférence entre 40°C et 45 °C, par exemple 42°C. L'élément de chauffage 12 est par exemple une résistance ménagée sur la plaque délimitant la chambre de collecte 10, produisant un chauffage par effet joule. Une augmentation locale et modérée de la température, au voisinage de la peau, favorise en effet une dilatation des capillaires sanguins, ce qui augmente la diffusion d'un gaz d'intérêt transcutané, à travers la peau. Par gaz d'intérêt, il est entendu un gaz dont on souhaite déterminer une concentration dans un corps humain ou animal vivant, et plus particulièrement dans le sang. Dans l'exemple décrit ci-dessous, de façon non limitative, le gaz d'intérêt est le dioxyde de carbone, dont on cherche à estimer une teneur dans le sang de l'utilisateur.

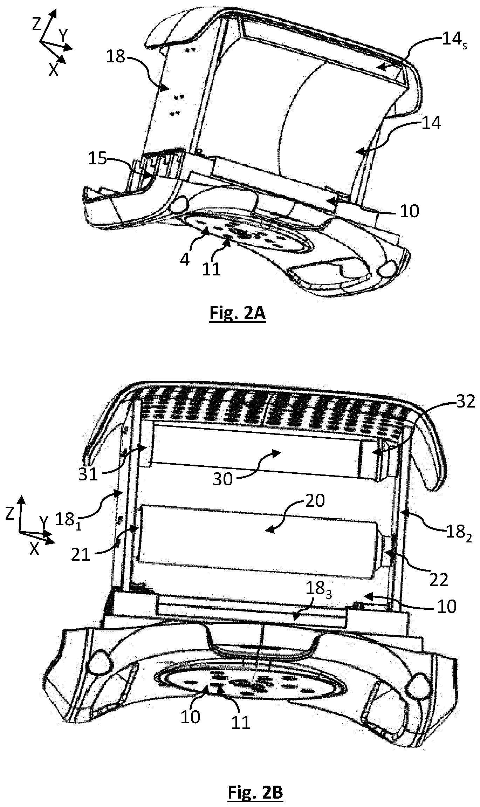

A l'intérieur du corps principal 2, la figure 2A montre un conduit 14, destiné à guider le $CO_2$ transcutané collecté par la chambre de collecte 10 jusqu'à une chambre de mesure 20, puis en aval de cette dernière, vers des sorties $14_s$, comme décrit ci-après.

Afin de faciliter le transport du $CO_2$ transcutané vers la chambre de mesure 20, le corps principal 2 comporte des ouvertures 15, destinées à admettre de l'air ambiant. L'air ambiant désigne l'air s'étendant autour du dispositif 1. Dans l'exemple représenté sur la figure 2B, les ouvertures 15 sont pratiquées à travers la face latérale 5. Sous l'effet de la chaleur dégagée au niveau de la chambre de collecte 10 sous l'action de l'élément de chauffage 12, un courant de convection thermique se forme. De ce fait, le $CO_2$ transcutané et l'air ambiant admis dans le corps principal forment un mélange gazeux s'éloignant de la face de contact 4 et se propageant en direction de la chambre de mesure 20, en direction de l'extrémité 6. Ainsi, l'air ambiant admis dans le corps principal 2 à travers les ouvertures 15 agit en tant que gaz vecteur et transporte le $CO_2$ transcutané à travers le corps principal, et notamment à travers la chambre de collecte 10, jusqu'à la chambre de mesure 20. Les ouvertures latérales, ainsi que le chauffage appliqué au niveau de la chambre de collecte forment un effet cheminée, qui constitue un "moteur" permettant une propagation du $CO_2$ à travers le corps principal 2 du dispositif, par convection.

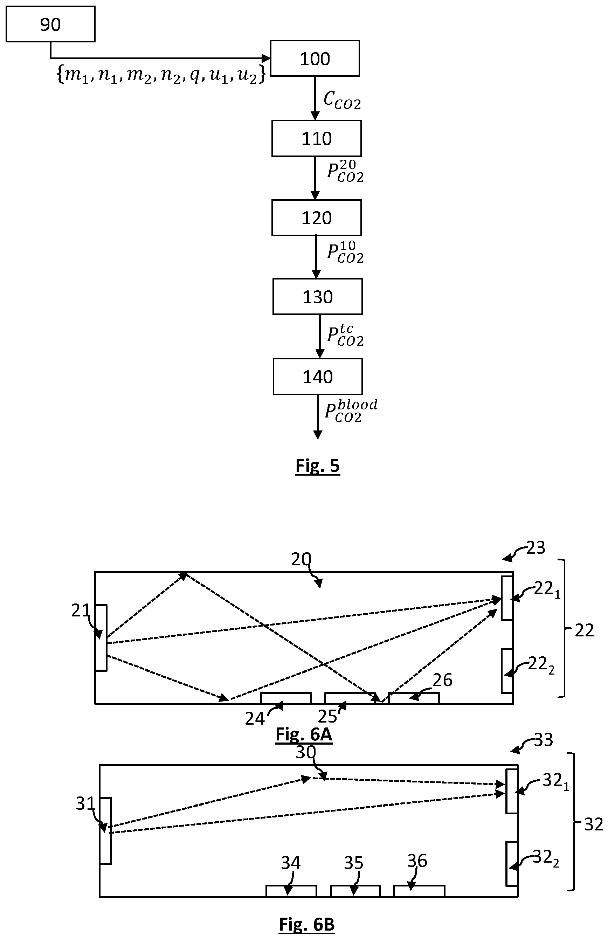

La chambre de mesure 20 est, dans cet exemple, disposée sensiblement parallèlement à la face de contact 4. La fonction de la chambre de mesure est d'estimer une concentration de $CO_2$ du mélange gazeux circulant dans le corps principal 2. A cette fin, la chambre de mesure 20 comporte un capteur de gaz 23. Plusieurs types de capteurs peuvent être utilisés à cette fin, par exemple des capteurs optiques ou des capteurs électrochimiques, ces derniers pouvant notamment être basés sur des oxydes métalliques (capteurs MOX). Les inventeurs ont estimé qu'il était préférable d'utiliser un capteur optique, et plus précisément un capteur infra-rouge. Un tel capteur ne nécessite pas de maintenance particulière, et est particulièrement compact, ainsi que peu onéreux. De plus, un tel capteur est très spécifique pour caractériser des liaisons chimiques. Il convient à la détection de molécules de petites tailles, par exemple le dioxyde de carbone. Les capteurs de gaz basés sur une absorption de la lumière infra-rouge sont connus, et sont usuellement désignés par le terme capteurs NDIR (Non Dispersive Infra Red). Ce type de capteur comporte une source de rayonnement infrarouge 21, émettant généralement dans une bande spectrale comprise entre 1 $\mu m$ et 20 $\mu m$. Il comporte également un photodétecteur 22, sensible au rayonnement infra-rouge. Le principe repose sur l'atténuation, par le gaz analysé, du rayonnement infra-rouge, émis par la source. La source infra-rouge 21 et le photodétecteur 22 forment le capteur de gaz 23, dont le principe de fonctionnement est détaillé en lien avec les figures 6A et 6B. Le photodétecteur 22 est par exemple une thermopile.

Le dispositif comporte de préférence une chambre de mesure auxiliaire 30. La chambre de mesure auxiliaire 30 comporte un capteur auxiliaire de $CO_2$ 33, fonctionnant de la même façon que le capteur 23 disposé dans la chambre de mesure 20. Il comporte une source de rayonnement infra-rouge 31 ainsi qu'un photodétecteur 32. Le capteur auxiliaire 33 est destiné à estimer une concentration en $CO_2$ dans l'air ambiant entourant le corps principal 2. Le recours à un tel capteur est préférable, mais non indispensable.

Le dispositif comporte des cartes électroniques $18_1$, $18_2$, $18_3$. L'ensemble des cartes électronique forment une unité de traitement et de calcul 18, configurée pour piloter les différents composants électroniques du dispositif, et traiter les informations issues par les différents détecteurs. L'unité de traitement et de calcul peut comporter des moyens de calculs, par exemple un microprocesseur, embarqué sur le dispositif ou situé à distance de ce dernier, en étant relié par une liaison filaire ou sans fil. L'unité de traitement et de calcul est également configurée pour mettre en oeuvre le procédé d'estimation de la teneur en $CO_2$ dissout du sang de l'utilisateur, dont les principales étapes sont schématisées sur la figure 5. A cet effet, l'unité de traitement peut comporter un ou plusieurs microprocesseurs.

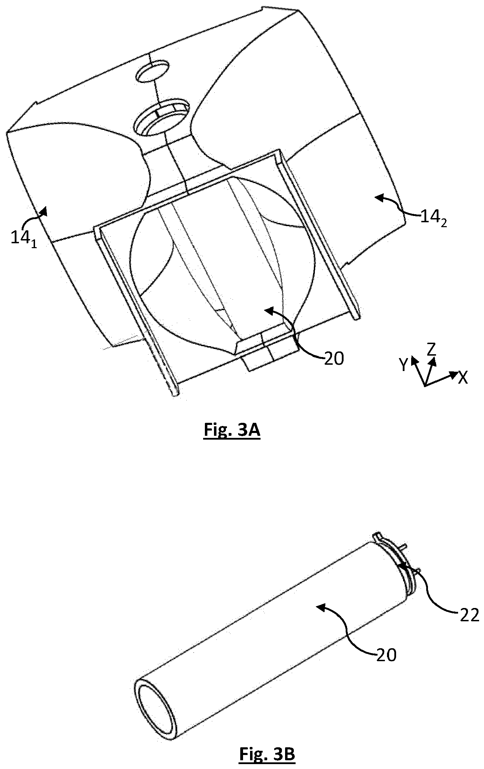

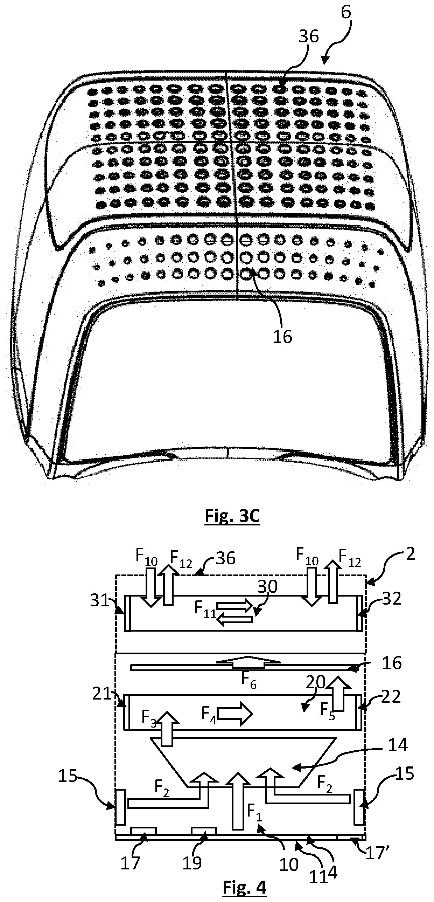

La figure 3A représente le conduit 14, qui permet de diriger le mélange gazeux jusqu'à la chambre de mesure 20. En aval de la chambre de mesure 20, le mélange gazeux est conduit vers des ouvertures s'évacuation 16, représentées sur la figure 3C. Plus précisément, le conduit 14 est divisé en deux conduits élémentaires $14_1$ et $14_2$, débouchant sur les ouvertures d'évacuation 16 disposées en aval des sorties $14_s$. La figure 3B montre la chambre de mesure 20, comportant notamment le photodétecteur 22.

Au niveau de l'extrémité distale 6, des ouvertures 36 sont ménagées, permettant un accès de l'air ambiant à une chambre de mesure auxiliaire 30. La chambre de mesure auxiliaire 30 est isolée du reste des composants du corps principal, de sorte qu'elle n'est exposée qu'à l'air ambiant s'étendant autour du corps principal 2. La chambre de mesure auxiliaire ne reçoit pas le $CO_2$ transcutané. La chambre de mesure auxiliaire 30 est optionnelle. Elle permet d'affiner la précision avec laquelle on peut estimer la pression partielle de $CO_2$ transcutanée dans le mélange gazeux circulant à travers la chambre de mesure 20.

La figure 4 schématise les courants fluidiques formés au niveau du dispositif. Le $CO_2$ transcutané est admis dans le dispositif par les ouvertures transversales 11 pratiquées dans la face de contact 4, et débouchant dans la chambre de collecte 10 (cf. flèche $F_1$). L'air ambiant est admis à travers les ouvertures 15 pratiquées dans la paroi latérale 5 (cf. flèches $F_2$). Le mélange gazeux comportant l'air ambiant et le $CO_2$ transcutané se propage vers la chambre de mesure 20 (cf. flèche $F_3$), à travers le conduit 14. Le mélange gazeux s'étend ensuite dans la chambre de mesure 20, entre la source de rayonnement infra-rouge 21 et le photodétecteur 22 (cf. flèche $F_4$), avant de sortir du corps principal 2 (cf. flèches $F_5$ et $F_6$).

Parallèlement à ce flux, l'air ambiant diffuse dans la chambre de mesure auxiliaire 30 à travers les ouvertures 36 (flèches $F_{10}$), et s'étend, par diffusion, à travers la chambre de mesure auxiliaire (flèches $F_{11}$ et flèches $F_{12}$).

L'unité de traitement et de calcul 18 collecte les mesures de la pression partielle de $CO_2$ dans la chambre de mesure 20 et dans la chambre de mesure auxiliaire 30. A partir de ces mesures, on estime successivement la pression partielle de $CO_2$ transcutané, ainsi que la pression partielle de $CO_2$ dans le sang, selon les étapes schématisées sur la figure 5.

Etape 100 : détermination de la concentration de $CO_2$ dans la chambre de mesure 20.

La mesure de la pression partielle de $CO_2$ dans la chambre de mesure 20 constitue une étape particulièrement importante. La figure 6A schématise la chambre de mesure 20. Elle définit un volume de mesure s'étendant entre une source de rayonnement infra-rouge 21 et le photodétecteur 22. La source et le détecteur sont des composants du capteur de $CO_2$ 23.

Le photodétecteur 22 comporte une voie de mesure $22_1$ et une voie de référence $22_2$. La voie de mesure $22_1$ est configurée pour détecter le rayonnement s'étant propagé à travers la chambre de mesure 20, dans une bande spectrale d'absorption qui correspond à l'espèce gazeuse que l'on souhaite analyser, en l'occurrence $CO_2$. Ainsi, la voie de mesure $22_1$ effectue une mesure de l'intensité du rayonnement transmis par le mélange gazeux dans une bande spectrale de détection centrée sur $\lambda_1$ = 4.26 $\mu$m. La voie de référence $22_2$ est configurée pour détecter le rayonnement s'étant propagé à travers la chambre de mesure 20 dans une bande spectrale de référence, dans laquelle l'absorption par le mélange gazeux est considérée comme négligeable. La bande spectrale de référence est par exemple centrée sur $\lambda_1$ = 3.91 $\mu$m. On note qu'à la longueur d'onde de détection $\lambda_1$ et à la longueur d'onde de référence $\lambda_2$, l'absorption du rayonnement par la vapeur d'eau peut être considérée comme égale. A défaut, la concentration d'humidité est prise en compte dans le modèle, comme décrit par la suite. Il en est de même de l'absorption de l'air, qui peut être considérée comme égale aux deux longueurs d'onde.

La chambre de mesure 20 comporte, de préférence, un capteur de température 24 ainsi qu'un capteur d'humidité 25 et un capteur de pression 26. La chambre de mesure auxiliaire 30 comporte, de préférence, un capteur de température auxiliaire 34 et un capteur d'humidité auxiliaire 35 et un capteur de pression auxiliaire 36.

Soient $U_0(\lambda_1)$ et $U(\lambda_1)$, les tensions mesurées aux bornes de la voie de mesure $22_1$ respectivement en l'absence de gaz et en présence de gaz dans la chambre de mesure 20. Soient $U_0(\lambda_2)$ et $U(\lambda_2)$, les tensions mesurées aux bornes de la voie de mesure $22_2$ respectivement en l'absence de gaz et en présence de gaz dans la chambre de mesure 20. Si $C_{CO2}$ est la concentration molaire de $CO_2$ à l'intérieur du mélange gazeux, on peut montrer que :

$$-ln\left[\frac{\frac{U(\lambda_1)}{U(\lambda_2)}}{\frac{U_0(\lambda_1)}{U_0(\lambda_2)}}\right] = [k_{CO2}(\lambda_1) - k_{CO2}(\lambda_2)]C_{CO2} + [k_{H2O}(\lambda_1) - k_{H2O}(\lambda_2)]C_{H2O}$$

$$+ [A_{air}(\lambda_1) - A_{air}(\lambda_2)] \quad (1)$$

où :

- $k_{CO2}$ est le coefficient d'atténuation du $CO_2$ ;
- $k_{H2O}$ est le coefficient d'atténuation de la vapeur d'eau $H_2O$ ;
- $A_{air}$ est l'atténuation de l'air ;
- $C_{H20}$ est la concentration molaire de $H_20$ (vapeur d'd'eau) dans le mélange gazeux Or, on peut considérer que $A_{air}(\lambda_1) = A_{air}(\lambda_2)$ et $k_{H2O}(\lambda_1) = k_{H2O}(\lambda_2)$ et que $k_{CO2}(\lambda_2) = 0$. L'expression (1) devient alors :

$$-ln\left[\frac{\frac{U(\lambda_1)}{U(\lambda_2)}}{\frac{U_0(\lambda_1)}{U_0(\lambda_2)}}\right] \approx k_{CO2}(\lambda_1)C_{CO2} \quad (2)$$

Ainsi, en première approche, la concentration de $CO_2$ dans la chambre de mesure 20 peut être estimée par une comparaison, prenant la forme d'un ratio, entre les signaux $U(\lambda_1)$ et $U(\lambda_2)$ respectivement générés par le photodétecteur de mesure et le photodétecteur de référence, sachant que le ratio $\frac{U_0(\lambda_1)}{U_0(\lambda_2)}$ est déterminé par une calibration préalablement à l'utilisation du dispositif, et est considéré comme constant durant l'utilisation du dispositif. On parle alors de mesure différentielle, car la concentration de $CO_2$ est estimée par une simple comparaison des signaux $U(\lambda_1)$ et $U(\lambda_2)$. L'expression (2) correspond à l'application d'un modèle linéaire, l'atténuation étant considérée comme variant linéairement en fonction de la concentration de $CO_2$.

Cependant, un tel modèle ne tient pas compte de plusieurs causes d'incertitudes ou de variabilités, en particulier le fait que :

- la concentration de vapeur d'eau $C_{H2O}$ dans le mélange gazeux est très supérieure à celle de $CO_2$ ;
- du fait de réflexions multiples à l'intérieur de la chambre de mesure, la distance parcourue par le rayonnement infra-rouge détecté, entre la source de rayonnement infra-rouge 21 et le photodétecteur 22, subit une variabilité. En effet, les parois internes de la chambre de mesure sont préférentiellement réfléchissantes. Par conséquent, les distances parcourues par les rayons lumineux émis par la source à travers l'enceinte sont variables. Du fait de la variabilité des chemins optiques parcourus à travers le gaz, l'atténuation s'étend dans une plage de variation. La figure 6A montre différents chemins optiques, schématisés par des flèches en pointillés. Le modèle linéaire tel que décrit en lien avec l'expression (2) ne tient pas compte d'une telle variabilité des chemins optiques.

- la bande spectrale de détection n'est pas parfaitement monochromatique, mais s'étend sur une certaine plage spectrale $\Delta\lambda_1$ autour de la longueur d'onde de détection $\lambda_1$. Or, dans la plage spectrale adressée, le coefficient d'atténuation du $CO_2$ $k_{CO2}$ ou de la vapeur d'eau $k_{H2O}$ varient.

Ces imperfections forment des sources de non-linéarité. C'est pourquoi les inventeurs considèrent que le modèle linéaire, tel qu'explicité dans les expressions (1) et (2) peut être amélioré. Ils proposent de modéliser les mesures résultant des photodétecteurs selon un modèle linéaire quadratique dépendant d'une puissance non-entière de la concentration des gaz. Selon un tel modèle, les mesures réalisées par les photodétecteurs de détection et de référence sont modélisées par l'expression suivante :

$$-ln\left[\frac{\frac{U(\lambda_1)}{U(\lambda_2)}}{\frac{U_0(\lambda_1)}{U_0(\lambda_2)}}\right] = m_1 C_{CO2}{}^{u_1} + n_1 C_{CO2}{}^{2u_1} + m_2 C_{H2O}{}^{u_2} + n_2 C_{H2O}{}^{2u_2} + q C_{CO2}{}^{u_1}.C_{H2O}{}^{u_2}(3)$$

où :

- $C_{CO2}$, et $C_{H2O}$ sont des concentrations de $CO_2$ et $H_2O$ dans le mélange gazeux, au niveau de la chambre de mesure 20; $C_{H2O}$ peut être obtenue en mettant en oeuvre un capteur d'humidité 25 dans la chambre de mesure 20 ;
- $m_1$, $n_1$, $m_2$, $n_2$, et $q$ sont les paramètres scalaires du modèle ;
- $u_1$, $u_2$ sont les réels, pouvant être non entiers, correspondant aux puissances des concentrations ;
- le ratio $U_0(\lambda_1)$ / $U_0(\lambda_2)$ est déterminé par une calibration préalablement à l'utilisation du dispositif.

Le terme bilinéaire $C_{CO2}{}^{u_1}.C_{H2O}{}^{u_2}$ peut être négligé, auquel cas $q = 0$.

La prise en compte d'un tel modèle présente l'avantage qu'il peut être inversé analytiquement de façon simple, sans nécessiter des moyens de calcul importants.

La concentration $C_{H2O}$ est déterminée à partir du capteur d'humidité 25. Il s'agit donc d'une grandeur considérée comme connue.

En effectuant les changements de variables suivants :

$$\gamma = m_2 C_{H2O}{}^{u_2} + n_2 C_{H2O}{}^{2u_2} + ln\left[\frac{\frac{U(\lambda_1)}{U(\lambda_2)}}{\frac{U_0(\lambda_1)}{U_0(\lambda_2)}}\right] \quad (4)$$

$$\beta = q C_{H2O}{}^{u_2} + m_1 \quad (5)$$

$$x = C_{CO2}{}^{u_1}(6)$$

L'expression (3) peut s'écrire :

$$n_1 x^2 + \beta x + \gamma = 0 \quad (7)$$

Il s'agit d'un polynôme du second degré, dont les solutions sont :

$$x = \frac{-\beta \pm \sqrt{\beta^2 - 4n_1\gamma}}{2n_1} \quad (8)$$

et

$$C_{CO2} = \sqrt[u_1]{x} \quad (9)$$

L'équation (8) donne deux valeurs possibles de x, dont une seule, généralement la plus faible, permet d'obtenir une concentration $C_{CO2}$ comprise dans une plage de valeurs prédéterminée. La plage de valeurs prédéterminée correspond à des valeurs auxquelles on peut s'attendre compte tenu de l'application visée.

Le recours au capteur d'humidité 25 est préférable, mais non pas nécessaire. Selon un modèle simplifié, les mesures peuvent être modélisées par :

$$-ln\left[\frac{\frac{U(\lambda_1)}{U(\lambda_2)}}{\frac{U_0(\lambda_1)}{U_0(\lambda_2)}}\right] = m_1 C_{CO2}{}^{u_1} + n_1 C_{CO2}{}^{2u_1} \quad (3')$$

En effectuant un changement de variable de type :

- 
$$\gamma = ln\left[\frac{\frac{U(\lambda_1)}{U(\lambda_2)}}{\frac{U_0(\lambda_1)}{U_0(\lambda_2)}}\right] \quad (4')$$

- 
$$\beta = m_1 \quad (5')$$

- 
$$x = C_{CO2}{}^{u_1} (6')$$

L'expression (3) peut s'écrire :

- 
$$n_1 x^2 + \beta x + \gamma = 0 \quad (7')$$

Il s'agit d'un polynôme du second degré, dont les solutions sont :

- 
$$x = \frac{-\beta \pm \sqrt{\beta^2 - 4n_1\gamma}}{2n_1} \quad (8')$$

Il est à noter que le recours à un tel modèle pour mesurer du $CO_2$, ou toute autre espèce gazeuse dont on connaît une bande spectrale d'absorption, est applicable indépendamment du dispositif et de l'application décrits dans cette demande de brevet. Ainsi, un tel modèle peut être utilisé pour le traitement de mesures basées sur l'atténuation d'un rayonnement infra-rouge par une espèce gazeuse, les mesures étant réalisées à la fois dans la bande spectrale d'absorption de l'espèce gazeuse considérée et dans une bande spectrale de référence dans laquelle l'absorption du rayonnement est considérée comme négligeable.

Etape 110 Détermination de la pression partielle de $CO_2$ dans la chambre de mesure 20.

Ayant déterminé la concentration molaire $C_{CO2}$, la pression partielle de $CO_2$ dans la chambre de mesure 20 est déterminée en appliquant la loi des gaz parfaits :

$$P_{CO2}^{20} = C_{CO2} R T^{20} \quad (10)$$

où :

- $T^{20}$ correspond à la température dans la chambre de mesure 20, mesurée à l'aide du capteur de température 24;

- $P_{CO2}^{20}$ est la pression partielle de $CO_2$ dans la chambre de mesure 20 ;
- $R$ est la constante des gaz parfaits.

<u>Etape 120.</u> Détermination de la pression partielle de $CO_2$ au niveau de la chambre de collecte 10.

A partir de la pression partielle de $CO_2$ dans la chambre de mesure 20, on détermine la pression partielle de $CO_2$ au niveau de la chambre de collecte 10. Pour cela on utilise l'expression suivante, reliant la pression totale mesurée dans la chambre de mesure 20, notée $P_{Total}^{20}$, ainsi que la pression totale mesurée dans la chambre de collecte 10, notée $P_{Total}^{10}$.

$$P_{CO2}^{10} = \frac{P_{Total}^{col}}{P_{Total}^{20}} \, P_{CO2}^{20} \quad (11)$$

où :

- $P_{Total}^{20}$ est la pression totale mesurée dans la chambre de mesure 20 par le capteur de pression 26.
- $P_{Total}^{col}$ est la pression totale mesurée dans la chambre de collecte 10 par un capteur de pression 19 disposé au niveau de la chambre de collecte 10, comme représenté sur la figure 4.

<u>Etape 130</u> Détermination de la pression transcutanée de $CO_2$.

Cette étape vise à déterminer la pression transcutanée de $CO_2$ à partir de la pression partielle de $CO_2$ dans la chambre de collecte 10. Il faut tenir compte de l'arrivée d'air par les ouvertures latérales 15. La chambre de collecte 10 comporte un capteur de température 17. Le dispositif comporte également un capteur 34 de température ambiante. Lorsque le dispositif comporte une chambre de mesure auxiliaire 30, le capteur de température ambiante est de préférence disposé dans cette dernière.

Selon une première approche, on néglige l'apport de $CO_2$ transporté par l'air ambiant admis à travers les ouvertures latérales 15. On se base alors sur une hypothèse selon laquelle le $CO_2$ circulant dans le dispositif est très majoritairement du $CO_2$ transcutané.

Selon cette première approche, la pression transcutanée $P_{CO_2}^{tc}$ de $CO_2$ dans la chambre de collecte 10 est :

$$P_{CO_2}^{tc}(T^{tc}) = \frac{T^{tc}}{T^{10}} \cdot g_{CO_2}^{tc/col} \cdot P_{CO_2}^{col}(T^{10}) \quad (12)$$

où :

- $P_{CO_2}^{tc}(T^{tc})$ est la pression transcutanée de $CO_2$ à la température transcutanée $T^{tc}$, c'est-à-dire à la température de la peau, et exprimée en mmHg. Cette température peut être mesurée par un capteur de température 17', comme décrit par la suite.
- $P_{CO_2}^{col}(T^{10})$ est la pression de $CO_2$ mesurée à température $T^{10}$ dans la chambre de collecte et exprimée en mmHg ;
- $T^{10}$ est une température mesurée dans la chamber de collecte par un capteur de température 17.

La notation $X(T^x)$ désigne le fait qu'une grandeur X est mesurée à la température $T^x$

La température de la peau $T^{tc}$ peut être supposée connue ou mesurée par un capteur de température de peau 17', permettant de connaître la température au niveau de la peau. Le capteur de température de peau peut également permettre de réguler le chauffage de la peau par rapport à une température de consigne, cette dernière étant de 42°C.

$g_{CO_2}^{tc/col}$ est un facteur de correction permettant de prendre en compte une dilution, dans la chambre de collecte 10, induite par l'air admis à travers les ouvertures 15. Ce facteur peut être déterminé par l'expression suivante :

$$g_{CO_2}^{tc/col} = \frac{k_{blood} \cdot \rho_{skin}^{blood} \cdot S_{skin} \cdot H_{b:air} + D_{air}}{k_{blood} \cdot \rho_{skin}^{blood} \cdot S_{skin} \cdot H_{b:air}} \quad (13)$$

- $k_{blood}$ est un coefficient de transfert massique, exprimé en $m.s^{-1}$, entre le sang et l'air ;

- $S_{blood}$ est la surface d'échange au niveau de l'interface sang/air

- $\rho_{skin}^{blood}$ est un ratio de perfusion sanguine à la surface de la peau :

$$\rho_{skin}^{blood} = \frac{S_{blood}}{S_{skin}} \quad (14)$$

- $S_{blood}$ est la surface de sang impliquée dans l'émission de $CO_2$ transcutanée;

- $S_{skin}$ est la surface de la peau impliquée dans l'émission de CO2 transcutanée : il s'agit de la surface de peau disposée en vis-à-vis du dispositif ;

- $H_{b:air}$ est un coefficient de partition sang/air correspondant également à une constant de Henry entre une concentration liquide et une concentration gazeuse ;

- $D_{air}$ est le débit d'air volumique à travers le dispositif ($m^3/s$), à travers les ouvertures latérales 15, établi comme décrit par la suite : Le coefficient de transfert massique sang/air et le coefficient de partition sang/air sont des grandeurs physiologiques données dans la littérature : Par exemple, le coefficient de transfert massique $k_{blood}$ peut être considéré égal à 23.1 $\mu$m/s. Le coefficient de partition sang/air $H_{b:air}$ peut être, par exemple, considéré comme égal à 0.59 à la température de 37°C. Le ratio $\rho_{skin}^{blood}$ est ajusté en fonction de la zone corporelle sur laquelle le dispositif 1 est appliqué.

La surface de la peau $S_{skin}$ disposée en vis-à-vis du dispositif est définie par la géométrie du dispositif.
Cette équation peut s'écrire :

$$g_{CO_2}^{tc/col} = 1 + \frac{D_{air}}{D_{CO_2}^{b:air}} \quad (16)$$

où $D_{CO_2}^{b:air}$ est le débit de diffusion de $CO_2$ à travers l'interface sang/air. Il peut être établi selon l'expression suivante :

$$D_{CO_2}^{b:air} = \rho_{skin}^{blood} \cdot S_{skin} \cdot k_{blood} \cdot H_{b:air} \quad (17)$$

et

$$D_{air}(T^{10}) = R.T^{10} \frac{\left(P_{air}^{out\,col}(T^{10}) - P_{air}^{amb}(T^{amb})\right)}{P_{air}^{out\,col}(T^{10}).R^{col-amb}} \quad (18)$$

- $P_{air}^{out\,col}$ .est la pression d'air mesurée dans la chambre de collecte 10;
- $P_{air}^{amb}$ est la pression d'air ambiant, mesuré par exemple dans la chambre auxiliaire 30;
- $R^{col-amb}$ est la résistance de Poiseuille entre la chambre de collecte et l'air ambiant ;
- $R$ est la constant des gaz parfaits.

La résistance de Poiseuille $R^{col-amb}$ peut être estimée en imposant un débit d'air dans le dispositif sur un banc gaz, et en mesurant les pressions $P_{air}^{out\,col}(T^{10})$ et $P_{air}^{amb}(T^{amb})$ grâce aux capteurs de pression du dispositif. On a alors:

$$R^{col-amb} = R.\,T^{10}\frac{\left(P_{air}^{out\,col}(T^{10}) - P_{air}^{amb}(T^{amb})\right)}{P_{air}^{out\,col}(T^{10}).\,D_{air}(T^{10})} \quad (19)$$

On peut montrer que :

$$[CO_2^{tc}](T^{tc}) \cong g_{CO_2}^{tc/col}.\,[CO_2^{10}](T^{10}) = g_{CO_2}^{tc/col}.\,[CO_2^{20}](T^{20}) \quad (20)$$

- $[CO_2^{tc}]$ est la concentration relative de $CO_2$ transcutané dans la chambre de collecte 10, exprimée en ppm ;

- $[CO_2^{10}]$ est la concentration totale de $CO_2$ dans la chambre de collecte 10, exprimée en ppm;

- $[CO_2^{20}]$ est la concentration de $CO_2$ dans la chambre de mesure 20 ;

- $[CO_2^{amb}]$ est la concentration de $CO_2$ dans l'air ambiant, mesuré dans la chambre auxiliaire 30, exprimée en ppm.

Selon une deuxième approche, on tient compte des effets géométriques et des variations de température sur l'air ambiant. La chambre de collecte 10 comporte un capteur de température 17. Le dispositif comporte également un capteur de température ambiante 34. Lorsque le dispositif comporte une chambre de mesure auxiliaire 30, le capteur de température ambiante est de préférence disposé dans cette dernière. Selon cette deuxième approche,

$$P_{CO_2}^{tc}(T^{tc}) = g_{CO_2}^{tc/col}.\,\frac{P_{CO_2}^{out\,col}(T^{10})}{P_{air}^{out\,col}(T^{10})}.\,P_{air}^{amb}(T^{amb}).\,\frac{T^{tc}}{T^{amb}}.\,\sqrt{\frac{S^{15}}{S^{11}}} \quad (21)$$

- $T^{amb}$ est la température de l'air ambiant. Elle est mesurée par un capteur de température 34, ce dernier pouvant être disposé au niveau de la chambre de mesure auxiliaire 30 lorsque le dispositif est équipé d'une telle chambre ;
- $T^{tc}$ est la température de la peau, par exemple égale à 42°C ;
- $T^{10}$ est la température dans la chambre de collecte, à travers de laquelle passe le $CO_2$ transcutané ; elle est mesurée par le capteur de température 17.
- $P_{air}^{amb}$ est la pression de l'air ambiant. Elle est mesurée par un capteur de pression 36 de l'air ambiant, ce dernier pouvant être disposé au niveau de la chambre de mesure auxiliaire 30 lorsque le dispositif est équipé d'une telle chambre ;
- $S^{15}$ est la surface des ouvertures latérales 15, à travers lesquelles l'air ambiant est admis dans la chambre de collecte 10 ;
- $S^{11}$ est la surface des ouvertures transversales 11, à travers lesquelles le $CO_2$ transcutané est admis dans la chambre de collecte 10.

Selon une troisième approche, on prend également en compte la concentration de $CO_2$ transporté par l'air ambiant, admis dans la chambre de collecte 10 à travers les ouvertures latérales 15. La concentration de $CO_2$ peut être prédéterminée, en prenant en compte une concentration prédéfinie, par exemple une concentration moyenne de $CO_2$ dans l'air. De façon avantageuse, la concentration de $CO_2$ dans l'air ambiant est mesurée à l'aide de la chambre de mesure auxiliaire 30, préalablement évoquée, ou par une autre méthode de mesure.

La chambre de mesure auxiliaire ne comporte que l'air ambiant. La figure 6B schématise la chambre de mesure auxiliaire. Le fonctionnement de la chambre de mesure auxiliaire 30 peut être similaire au fonctionnement de la chambre de mesure 20. En particulier, la source auxiliaire de rayonnement infra-rouge 31 émet un rayonnement dans une bande spectrale d'émission comprise entre 1 μm et 20 μm. Le photodétecteur auxiliaire 32 comporte une voie de mesure $32_1$ centrée sur 4.28 μm et une voie de référence $32_2$ centrée sur 3.91 μm. La chambre de mesure auxiliaire permet d'obtenir des mesures, pouvant être modélisées selon un modèle non linéaire tel que décrit en lien avec l'étape 100 :

$$-ln\left[\frac{\frac{U'(\lambda_1)}{U'(\lambda_2)}}{\frac{U'_0(\lambda_1)}{U'_0(\lambda_2)}}\right] = m'_1 {C_{CO2}^{amb}}^{u_1} + n'_1 {C_{CO2}^{amb}}^{2u_1} + m'_2 {C_{H2O}^{amb}}^{u_2} + n'_2 {C_{H2O}^{amb}}^{2u_2} + q' {C_{CO2}^{amb}}^{u_1} . {C_{H2O}^{amb}}^{u_2} \quad (22)$$

où :

- $C_{CO2}^{amb}$ est la concentration de $CO_2$ dans l'air ambiant ;

- $C_{H2O}^{amb}$ est la concentration d'$H_2O$ dans l'air ambiant, cette dernière pouvant être mesurée par un capteur d'humidité auxiliaire 35 disposé dans la chambre de mesure auxiliaire 30.

- $U'_0(\lambda_1)$ et $U'(\lambda_1)$ sont les signaux, par exemple les tensions, mesurés aux bornes de la voie de mesure $32_1$ respectivement en l'absence de gaz et en présence de gaz dans la chambre de mesure auxiliaire 30.

- $U'_0(\lambda_2)$ et $U'(\lambda_2)$ sont les signaux, par exemple les tensions, mesurés aux bornes du de la voie de référence $32_2$ respectivement en l'absence de gaz et en présence de gaz dans la chambre de mesure auxiliaire 30.

- $m'_1$, $n'_1$, $m'_2$, $n'_2$, et $q'$ sont les paramètres scalaires du modèle correspondant au capteur auxiliaire 33;

- $u'_1$, $u'_2$ sont les réels, pouvant être non entiers, correspondant aux puissances des concentrations.

Les valeurs $m'_1$, $n'_1$, $m'_2$, $n'_2$, $q'$, $u'_1$ et $u'_2$ peuvent être déterminés au cours d'une phase de calibration, telle que celle décrite dans l'étape 90.

La prise en compte de la concentration de $CO_2$ dans l'air ambiant est considérée comme celle susceptible de conduire à l'estimation la plus précise de la pression partielle de $CO_2$ dans le sang.

Selon cette troisième approche, on soustrait la contribution en $CO_2$ de l'air ambiant ayant été admis dans la chambre de collecte 10 à travers les ouvertures latérales.

Ainsi :

$$[CO_2^{tc}](T^{tc}) = g_{CO_2}^{tc/col} . [CO_2^{10}](T^{10})$$

$$- \frac{D_{air}}{k_{blood} . \rho_{skin}^{blood} . S_{skin} . H_{b:air}} . \sqrt{\frac{S^{15}}{S^{11}}} . [CO_2^{amb}](T^{amb}) \quad (25)$$

Or,

$$[CO_2^{10}](T^{10}) = [CO_2^{20}](T^{20}) \quad (26)$$

Donc :

$$[CO_2^{tc}](T^{tc}) = g_{CO_2}^{tc/col} . [CO_2^{20}](T^{20}) - \frac{D_{air}(T^{10})}{D_{CO_2}^{b:air}} . \sqrt{\frac{S^{15}}{S^{11}}} . [CO_2^{amb}](T^{amb}) \quad (27)$$

$D_{CO_2}^{b:air}$ est le débit volumique de diffusion du $CO_2$ à travers l'interface sang / air. Si on ne tient compte que du changement de phase sang/air, nous pouvons utiliser l'expression:

$$D_{CO_2}^{b:air} = \rho_{skin}^{blood} . S_{skin} . k_{blood} . H_{b:air} \quad (28)$$

Si nous souhaitons aussi tenir compte de l'effet de la peau sur la diffusion du gaz, nous pouvons introduire le coefficient binaire de diffusion de la peau $D_{skin}^{bin}$ ($m.s^{-1}$). Il définit la vitesse de diffusion du gaz carbonique à travers la peau.

Dans ce cas, l'expression de $D_{CO_2}^{b:air}$ devient:

$$D_{CO_2}^{b:air} = \rho_{skin}^{blood} . S_{skin} . K_{CO_2}^{blood\ to\ col} \quad (29)$$

$K_{CO_2}^{blood\ to\ col}$ est le coefficient de transfert de masse total entre le sang et l'air combinant le changement de phase et la diffusion à travers la peau :

$$\frac{1}{K_{CO_2}^{blood\ to\ col}} = \frac{1}{k_{blood} H_{b:air}} + \frac{1}{D_{skin}^{bin}} \quad (30)$$

$D_{air}(T^{10})$ est le débit volumique d'air admis à travers les ouvertures latérales 15 dans la chambre de collecte 10, tel que :

$$D_{air}(T^{10}) = R . T^{10} \frac{\left(P_{air}^{out\ col}(T^{10}) - P_{air}^{amb}(T^{amb})\right)}{P_{air}^{out\ col}(T^{10}) . R^{col-amb}} \quad (31)$$

On peut également écrire :

$$[CO_2^{tc}](T^{tc}) = g_{CO_2}^{tc/col} . [CO_2^{10}](T^{10}) - \frac{D_{air}(T^{10})}{D_{CO_2}^{skin}} . \sqrt{\frac{S^{15}}{S^{11}}} . [CO_2^{amb}](T^{amb}) \quad (32)$$

Or,

$$[CO_2^{10}](T^{10}) = [CO_2^{20}](T^{20}) \quad (33)$$

Donc

$$[CO_2^{tc}](T^{tc}) = g_{CO_2}^{tc/col} . [CO_2^{20}](T^{20}) - \frac{D_{air}(T^{10})}{D_{CO_2}^{skin}} . \sqrt{\frac{S^{15}}{S^{11}}} . [CO_2^{amb}](T^{amb}) \quad (34)$$

avec :

$$g_{CO_2}^{tc/col} = 1 + \frac{D_{air}(T^{10})}{D_{CO_2}^{b:air}},$$

comme précédemment défini. $D_{CO_2}^{skin}$ est défini en lien avec l'expression (48).

Pour la pression partielle, nous avons la relation :

$$P_{CO_2}^{tc}(T^{tc}) = \frac{T^{tc}}{T^{10}} \left[ g_{CO_2}^{tc/col} . P_{CO_2}^{10}(T^{10}) - \frac{D_{air} P_{CO_2}^{amb}(T^{10})}{k_{blood} . \rho_{skin}^{blood} . S_{skin} . H_{b:air}} \right] \quad (35)$$

ou :

$$P_{CO_2}^{tc}(T^{tc}) = \frac{T^{TC}}{T^{10}}\left[P_{CO_2}^{10}(T^{10}) + \frac{D_{air}}{D_{CO_2}^{b:air}}\cdot\left[P_{CO_2}^{10}(T^{10}) - P_{CO_2}^{amb}(T^{10})\right]\right] \quad (36)$$

Etape 140 : estimation de la pression partielle du $CO_2$ dans le sang.

Cette étape vise à déterminer une fonction $f$, de telle sorte que :

$$P_{CO2}^{blood} = f(P_{CO2}^{tc}) \quad (40)$$

où :

-  $P_{CO2}^{tc}$ est la pression partielle de $CO_2$ transcutanée lors de l'étape 130;

-  $P_{CO2}^{blood}$ est la pression partielle de $CO_2$ dans le sang, ce qui est la grandeur physiologique que le procédé visé à estimer.

Durant cette étape, on suppose, selon une première variante, que la pression partielle de $CO_2$ dans la chambre de collecte $P_{CO2}^{10}$ correspond à la pression partielle de $CO_2$ transcutanée $P_{CO2}^{tc}$.

La pression partielle de $CO_2$ dans le sang peut être estimée par une formule empirique donnée dans J.W. Severinghaus "The current status of transcutaneous blood gas analysis and monitoring", Blood Gas News, vol. 7, n° 2, pp 4-9, 1998.

$$P_{CO2}^{blood}(37°C) = \frac{P_{CO2}^{blood}(T^{tc}) - 4}{e^{(0.046\,(T^{tc}-37))}} \quad (41)$$

où $T^{tc}$ est une température mesurée par le capteur de température 17'.

Pour utiliser la formule (41), les températures sont exprimées en °C et les pressions sont exprimées en mmHg.

D'autres méthodes sont applicables pour estimer $P_{CO2}^{blood}$ à partir de $P_{CO2}^{tc}$. Selon une première variante, on peut se baser sur une modélisation du flux de $CO_2$ transcutané $\Phi_{CO2}$ diffusant à travers la peau.

Le flux gazeux de $CO_2$ à travers la peau $\Phi_{CO_2}^{g,blood\ to\ collection}$ est modélisé par :

Une équation de diffusion en phase gazeuse à travers la peau (effet de la peau):

$$\Phi_{CO_2}^{g,blood\ to\ collection} = S_{blood}\cdot D_{skin}^{bin}\cdot\left(C_{CO_2}^{g,blood} - C_{CO_2}^{col}\right) \quad (40)$$

$D_{skin}^{bin}$ est un coefficient binaire de diffusion de la peau ($m.s^{-1}$) correspondant au coefficient de transfert de masse du gaz carbonique à travers la peau. Il est déterminé soit expérimentalement sur des biopsies de peau, soit numériquement par apprentissage des paramètres du modèle sur des personnes pour lesquelles des mesures de référence de la concentration de gaz carboniques sont réalisées par une autre technique de mesure de la concentration du gaz carbonique dans le sang (prélèvement sanguin, mesure dans l'air exhalé, mesure de la pression transcutanée par capteurs électrochimiques,...) et où les paramètres sont ajustés pour faire correspondre les pressions estimées par le dispositif aux pressions de référence mesurées. $C_{CO_2}^{g,blood}$ est une concentration molaire de $CO_2$ en phase gazeuse dans le sang ($mol.mm^{-3}$) en équilibre avec la concentration en phase liquide suivant la loi de Henry :

$$C_{CO_2}^{g,blood} = \sigma_{CO_2}\cdot P_{CO_2}^{blood} \quad (41)$$

$P_{CO_2}^{blood}$ est la pression partielle de CO2 dans le sang, exprimée en mmHg (millimeters de mercure), et $\sigma_{CO_2}$ est le coefficient de solubilité d'Ostwald qui peut être tel que :

$$\sigma_{CO_2} = 3.3 \ x \ 10^{-2} \frac{mM}{mmHg} \quad (42)$$

$C_{CO_2}^{col,*}$ est la concentration molaire de $CO_2$ qu'il y aurait à l'équilibre dans la chambre de collecte si le gaz carbonique n'était pas éliminé par convection. C'est aussi la concentration qui existe au niveau de la chambre de collecte contre la peau. C'est la concentration transcutanée correspondant à la pression transcutanée. $C_{CO_2}^{col}$ est la concentration molaire de $CO_2$ dans la chambre de collecte 10.

Du fait de l'évacuation, par convection, du $CO_2$ de la chambre de collecte, la concentration $C_{CO_2}^{col}$ est différente de la concentration $C_{CO_2}^{col,*}$ .

L'équation de transfert de masse entre la phase liquide et la phase gazeuse liée uniquement au changement de phase s'écrit:

$$\Phi_{CO_2}^{g,blood \ to \ collection} = -S_{blood}k_{blood}H_{b:air}.\left(C_{CO_2}^{col} - C_{CO_2}^{col,*}\right) \quad (43)$$

En combinant les deux équations de transfert de masse, on obtient :

$$\Phi_{CO_2}^{blood \ to \ collection} = -S_{blood}K_{CO_2}^{blood \ to \ col}.\left(C_{CO_2}^{col} - C_{CO_2}^{g,blood}\right) \quad (44)$$

Où $K_{CO_2}^{blood \ to \ col}$ est le coefficient de transfert de masse total combinant le changement de phase et la diffusion à travers la peau:

$$\frac{1}{K_{CO_2}^{blood \ to \ col}} = \frac{1}{k_{blood}H_{b:air}} + \frac{1}{D_{skin}^{bin}} \quad (45)$$

On peut déduire de ce qui précède, l'expression :

$$P_{CO_2}^{blood}(T^{tc}) = \frac{T^{tc}}{T^{10}}.g_{CO_2}^{blood/col}.P_{CO_2}^{10}(T^{10}) \quad (46)$$

$g_{CO_2}^{blood/col}$ est un facteur de correction permettant de corriger l'effet de dilution dans la chambre de collecte ainsi que l'effet de diffusion à travers la peau et l'effet du changement de phase du sang vers l'air.

$$g_{CO_2}^{blood/col} = \frac{\rho_{skin}^{blood}.S_{11}.K_{CO_2}^{blood \ to \ col} + D_{air}}{\rho_{skin}^{blood}.S_{11}.K_{CO_2}^{blood \ to \ col}} \quad (47)$$

Il vient alors

$$P_{CO_2}^{blood}(T^{tc}) = P_{CO_2}^{tc}(T^{tc}) + \frac{T^{tc}}{T^{10}} \cdot \frac{D_{air}}{D_{CO_2}^{skin}} P_{CO_2}^{10}(T^{10}) \quad (48)$$

Où :

- $D_{CO_2}^{skin}$ est un débit volumique de diffusion du $CO_2$ à travers la peau, tel que :

$$D_{CO_2}^{skin} = \rho_{skin}^{blood} . S_{skin} . D_{skin}^{bin} \quad (49)$$

- $D_{air}(T^{10})$ est un débit volumique de l'air ambiant dans la chambre de collecte 10, tel que :

$$D_{air}(T^{10}) = R . T^{10} \frac{\left( P_{air}^{out\ col}(T^{10}) - P_{air}^{amb}(T^{10}) \right)}{P_{air}^{out\ col}(T^{10}) . R^{col-amb}} \quad (50)$$

- $P_{CO_2}^{amb}(T^{10})$ est la pression du $CO_2$, dans la chambre de collecte, venant de l'air ambiant, telle que :

$$P_{CO_2}^{amb}(T^{10}) = \frac{T^{10}}{T^{amb}} . P_{CO_2}^{amb}(T^{amb}) \quad (51)$$

- $R^{col-amb}$ correspond à la résistance fluidique entre l'air ambiant et la chambre de collecte.

La pression partielle $P_{CO2}^{blood}$ estimée par les formules qui précèdent est estimée à la température de la chambre de collecte 10, qui est plus élevée que 37°C. Or, le sang est à une température de 37°C. Il faut donc effectuer une correction de façon à obtenir une estimation de $P_{CO2}^{blood}$. Une telle correction peut être effectuée selon J.W. Severinghaus "The current status of transcutaneous blood gas analysis and monitoring", Blood Gas News, vol. 7, n° 2, pp 4-9, 1998, ce dernier déterminant une fonction de transfert *f*, telle que :

$$f = \frac{P_{CO2}^{blood}(T^{10})}{P_{CO2}^{blood}(T=37°C)} = e^{0.046[T^{10}-37]} \quad (52)$$

- où $T^{10}$ est la température dans la chambre de collecte 10

Ainsi :

$$P_{CO_2}^{blood}(37°C) = \frac{P_{CO_2}^{blood}(T^{10})}{exp\left(0.046[T^{10}-37]\right)} \quad (53)$$

La pression partielle issue de l'équation précédente est exprimée en mmHg.

Selon une deuxième variante, on prend en compte le $CO_2$ présent dans l'air ambiant admis dans la chambre de collecte à travers les ouvertures transversales. Les équations explicitées en lien avec la première variante deviennent :

$$P_{CO_2}^{tc}(T^{tc}) = \frac{T^{tc}}{T^{10}} \left[ g_{CO_2}^{blood/col} . P_{CO_2}^{col}(T^{10}) - \frac{D_{air} . P_{CO_2}^{amb}(T^{10})}{K_{CO_2}^{blood\ to\ col} . \rho_{skin}^{blood} . S_{skin}} \right] \quad (55)$$

$g_{CO_2}^{blood/col}$ est un facteur de correction permettant de corriger l'effet de dilution dans la chambre de collecte ainsi que l'effet de diffusion à travers la peau et l'effet du changement de phase du sang vers l'air.

$$g_{CO_2}^{blood/col} = \frac{\rho_{skin}^{blood} . S_{skin} . K_{CO_2}^{blood\ to\ col} + D_{air}}{\rho_{skin}^{blood} . S_{skin} . K_{CO_2}^{blood\ to\ col}} \quad (56)$$

soit

$$g_{CO_2}^{blood/col} = 1 + \frac{D_{air}}{\rho_{skin}^{blood} . S_{skin} . K_{CO_2}^{blood\ to\ col}} \quad (57)$$

donc

$$g_{CO_2}^{blood/col} = 1 + \frac{D_{air}}{\rho_{skin}^{blood} . S_{skin}} \left[ \frac{1}{k_{blood} H_{b:air}} + \frac{1}{D_{skin}^{bin}} \right] \quad (58)$$

On en déduit que :

$$P_{CO_2}^{blood}(T^{tc}) = P_{CO_2}^{tc}(T^{tc}) + \frac{T^{tc}}{T^{10}} . \frac{D_{air}}{D_{CO_2}^{skin}} \left[ P_{CO_2}^{tc}(T^{10}) - P_{CO_2}^{amb}(T^{10}) \right] \quad (59)$$

- $D_{CO_2}^{skin}$ est un débit volumique de diffusion du $CO_2$ à travers la peau, tel que :

$$D_{CO_2}^{skin} = \rho_{skin}^{blood} . S_{skin} . D_{skin}^{bin} \quad (60)$$

- $D_{air}(T^{10})$ est un débit volumique de l'air ambiant dans la chambre de collection 10, tel que :

$$D_{air}(T^{10}) = R . T^{10} \frac{\left( P_{air}^{out\ col}(T^{10}) - P_{air}^{amb}(T^{amb}) \right)}{P_{air}^{out\ col}(T^{10}) . R^{col-amb}} \quad (61)$$

- $P_{CO_2}^{amb}(T^{10})$ est la pression du $CO_2$, dans la chambre de collecte, venant de l'air ambiant, telle que :

$$P_{CO_2}^{amb}(T^{10}) = \frac{T^{10}}{T^{amb}} . P_{CO_2}^{amb}(T^{amb}) \quad (62)$$

$$P_{CO2}^{blood} = \frac{P_{CO2}^{10}}{P_{air}^{10}} \left[ P_{air}^{amb} \frac{T^{10}}{T^{amb}} \sqrt{\frac{S^{15}}{S^{11}}} + \frac{(P_{air}^{10} - P_{air}^{amb})}{R^{col-amb} S_{skin} \sigma_{CO2} D} \right] \quad (63)$$

En prenant en compte le modèle de Severinghaus :

$$P_{CO_2}^{blood}(37°C) = \frac{P_{CO_2}^{blood}(T^{10})}{exp(0.046[T^{10} - 37])} \quad (64)$$

Dans l'expression qui précède, $T^{10}$ est exprimé en °C.

<u>Etape 90</u> : Calibration du modèle

L'étape 100 nécessite de disposer des paramètres du modèle $m_1$, $n_1$, $m_2$, $n_2$, et $q$ ainsi que les valeurs des puissances $u_1$, $u_2$. Pour cela, préalablement à la mise en oeuvre du procédé, on met en oeuvre une calibration, visant à déterminer les valeurs des paramètres et des puissances. La calibration peut être une calibration supervisée, en utilisant un nombre $N_{cal}$ d'échantillons de calibration dont la teneur en $CO_2$ est connue.

Les paramètres et puissances sont estimés en minimisant une fonctionnelle J, telle que :

$$J = \sum_{i=1}^{N_{cal}} \left[ -ln \left[ \frac{\frac{U_i(\lambda_1)}{U_i(\lambda_2)}}{\frac{U_0(\lambda_1)}{U_0(\lambda_2)}} \right] - A_i \right]^2 \quad (70)$$

avec

$$A_i = m_1 C_{i_{CO2}}{}^{u_1} + n_1 C_{i_{CO2}}{}^{2u_1} + m_2 C_{i_{H2O}}{}^{u_2} + n_2 C_{i_{H2O}}{}^{2u_2} + q C_{i_{CO2}}{}^{u_1}. C_{i_{H2O}}{}^{u_2}$$

Autrement dit,

$$\{m_1, n_1, m_2, n_2, q, u_1, u_2\} = argmin\, J = argmin \left( \sum_{i=1}^{N_{cal}} \left[ -ln \left[ \frac{\frac{U_i(\lambda_1)}{U_i(\lambda_2)}}{\frac{U_0(\lambda_1)}{U_0(\lambda_2)}} \right] - A_i \right]^2 \right) \quad (71)$$

D'une façon générale, il s'agit de déterminer les paramètres pour lesquels compte tenu de la concentration connue de $CO_2$, l'application du modèle se rapproche le plus des grandeurs mesurées.

Le modèle étant non-linéaire, l'ensemble des paramètres $\{m_1, n_1, m_2, n_2, q, u_1, u_2\}$ minimisant $J$ peut être déterminé en mettant en oeuvre un algorithme de Levenberg Marquardt.

Un terme de régularisation peut être introduit pour rendre l'estimation des paramètres plus robuste aux erreurs de mesure et aux approximations sur le modèle :

$$\{m_1, n_1, m_2, n_2, q, u_1, u_2\} = argmin\, J$$

$$= argmin \left( \frac{1}{N_{cal}} \sum_{i=1}^{N_{cal}} \left[ -ln \left[ \frac{\frac{U_i(\lambda_1)}{U_i(\lambda_2)}}{\frac{U_0(\lambda_1)}{U_0(\lambda_2)}} \right] - A_i \right]^2 \right.$$

$$\left. + \omega \frac{1}{dim(\{m_1, n_1, m_2, n_2, q, u_1, u_2\})} \|\{m_1, n_1, m_2, n_2, q, u_1, u_2\}\|^2 \right) \quad (72)$$

où :

- $dim(\{m_1, n_1, m_2, n_2, q, u_1, u_2\})$ représente la dimension du vecteur de paramètres, qui vaut dans ce cas 7

- $\|\{m_1, n_1, m_2, n_2, q, u_1, u_2\}\|$ représente la norme du vecteur de paramètres. Dans le cas d'une norme quadratique :

$$\|\{m_1, n_1, m_2, n_2, q, u_1, u_2\}\|^2 = m_1{}^2 + n_1{}^2 + m_2{}^2 + n_2{}^2 + q^2 + u_1{}^2 + u_2{}^2$$

Le paramètre $\omega$ peut être choisi a priori, ou déterminé automatiquement par des techniques de type validation croisée.

Pour forcer le modèle à être proche d'un modèle linéaire quadratique standard on peut imposer que les puissances soient proches de 1. Dans ce cas :

$$\{m_1, n_1, m_2, n_2, q, u_1, u_2\} = argmin\, J$$

$$= argmin\left( \frac{1}{N_{cal}} \sum_{i=1}^{N_{cal}} \left[ -ln\left[ \frac{\frac{U_i(\lambda_1)}{U_i(\lambda_2)}}{\frac{U_0(\lambda_1)}{U_0(\lambda_2)}} \right] - A_i \right]^2 \right.$$

$$+ \omega \frac{1}{dim(\{m_1, n_{1-1}, m_2, n_2 - 1, q, u_1, u_2\})} \|\{m_1, n_1 - 1, m_2, n_2$$

$$\left. -1, q, u_1, u_2\}\|^2 \right) \quad (73)$$

Le facteur de correction de la dilution $g_{CO_2}^{tc/col}$ peut être estimé en réalisant plusieurs acquisitions d'apprentissage sur des personnes volontaires sur lesquelles des mesures de référence soit de la pression transcutanée, soit de la pression artérielle sont réalisées simultanément avec la mesure du dispositif lors de tests d'apprentissage. On estime alors la valeur moyenne du facteur $g_{CO_2}^{tc/col}$ à appliquer pour que les valeurs estimées de la pression transcutanée $P_{CO_2}^{tc}(T^{tc})$ s'approchent le mieux possible des valeurs de référence mesurées de la pression transcutanée ou que les valeurs estimées de la pression artérielle $P_{CO_2}^{blood}(37°C)$ s'approchent le mieux possibles des valeurs de référence mesurées de la pression artérielle.

Exemple de calibration en phase gazeuse

Au cours d'un premier exemple, on a calibré le modèle non linéaire explicité dans l'expression (3), selon l'étape 90, en alimentant la chambre de mesure à l'aide d'un gaz dont la concentration en $CO_2$ suivait, au cours de chaque essai, des paliers de concentrations, respectivement égaux à 0, 0.2, 0.4, 0.8, 1.6 et 3.2 mol/m$^3$. 12 essais ont été réalisés, en faisant varier la température $T^{20}$ de la chambre de mesure 20, ainsi que la concentration en valeur d'eau $C_{H2O}$, ainsi que la température $T^{10}$ dans la chambre de collecte 10

Le tableau 1 montre les paramètres expérimentaux pris en compte au cours de chaque essai.

Tableau 1

| Essai | $C_{H2O}$ (mol/m$^3$) | $T^{10}$ (°C) | $T^{20}$(°C) |
|---|---|---|---|
| 1 | 0.126 | 30.1 | 30.3 |
| 2 | 0.301 | 39.7 | 32.0 |
| 3 | 0.365 | 37 | 32.4 |
| 4 | 0.364 | 39.7 | 31.8 |
| 5 | 0.691 | 40.1 | 32.2 |
| 6 | 0.428 | 30.2 | 30.2 |
| 7 | 0.623 | 37.1 | 32.0 |
| 8 | 0.898 | 40.1 | 31.5 |

(suite)

| Essai | $C_{H2O}$ (mol/m³) | $T^{10}$ (°C) | $T^{20}$(°C) |
|---|---|---|---|
| 9 | 0.863 | 37.2 | 31.6 |
| 10 | 0.609 | 30.3 | 30.3 |
| 11 | 0.753 | 30.1 | 30.2 |
| 12 | 1.160 | 37.1 | 32.3 |

On a estimé les paramètres et les puissances du modèle en considérant trois modèles :

-  modèle A : sans prise en compte de la vapeur d'eau, tel que décrit en lien avec l'expression (3') ;
-  modèle B : modèle de l'expression (3) sans prise en compte du terme bilinéaire, ce qui revient à considérer $q = 0$ ;
-  modèle C : modèle de l'expression (3).

Le Tableau 2 présente les paramètres du modèle obtenus suite à la minimisation décrite dans l'étape 90.

Tableau 2

| Paramètres | Modèle A | Modèle B | Modèle C |
|---|---|---|---|
| $ln()$ | 1.08 | 0.99 | 1.07 |
| $m_1$ | 0.29 | 0.29 | 0.29 |
| $u_1$ | 0.61 | 0.59 | 0.57 |
| $n_1$ | 0.051 | 0.048 | 0.044 |
| $m_2$ | | 0.0040 | 0.0015 |
| $u_2$ | | 0.12 | 2.45 |
| $n2$ | | 0.065 | 0.00001 |
| $q$ | | | 0.00028 |

Dans le tableau 2, le paramètre $ln()$ correspond à $ln\left[\frac{U_0(\lambda_1)}{U_0(\lambda_2)}\right]$. Cette valeur est mesurée avant d'effectuer les essais, en l'absence de $CO_2$ dans la chambre de mesure.

Les trois modèles ont été comparés en comparant, lors de chaque essai, et pour chaque concentration de $CO_2$, les valeurs mesurées avec les valeurs réelles des concentrations. On a calculé, pour chaque modèle, une racine carrée de l'écart quadratique moyen (RMSE) ainsi que le coefficient de détermination ($R^2$). Le tableau 3 rassemble les résultats obtenus.

Tableau 3

| Modèle | RMSE | $R^2$ |
|---|---|---|
| A | 0.0180 | 0.982 |
| B | 0.0140 | 0.989 |
| C | 0.0133 | 0.990 |

Les résultats présentés dans le tableau 3 montrent que le modèle C, tel que décrit en lien avec l'expression (3) présente le coefficient RMSE le plus faible et le coefficient de détermination le plus élevé. Il est donc optimal. On observe également que la prise en compte de la concentration en vapeur d'eau (modèles B et C) permet d'améliorer les performances.

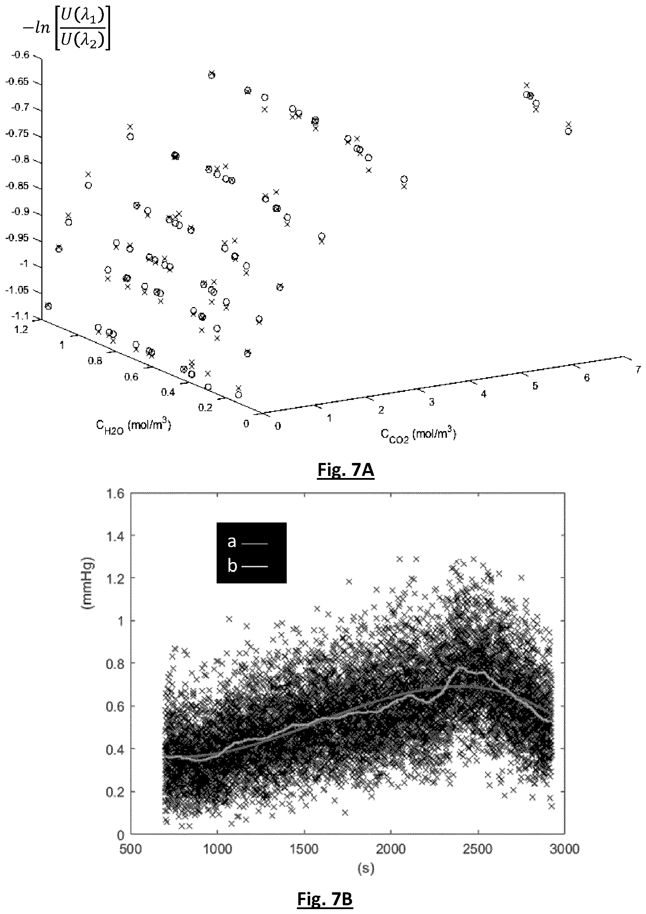

La figure 7A montre, pour chaque essai, les valeurs de exactes de $-ln\left[\frac{U(\lambda_1)}{U(\lambda_2)}\right]$ mesurées (croix) et les valeurs de $-ln\left[\frac{U(\lambda_1)}{U(\lambda_2)}\right]$ calculées (cercles) en mettant en oeuvre le modèle C. On observe la cohérence entre les valeurs mesurées et calculées, ce qui atteste de la pertinence du modèle.

Calibration en phase liquide

Afin d'estimer les constantes du modèle fluidique, il est aussi possible de réaliser une calibration en utilisant des mélanges dans un liquide de référence comme l'eau, pour des concentrations de $CO_2$ connues. La dilution du $CO_2$ afin de réaliser les mélanges peut être accélérée en utilisant un bulleur et un mélangeur. Différentes techniques permettent de contrôler la concentration du $CO_2$ dissout dans un liquide, par exemple de l'eau, par exemple en utilisant une sonde dédiée, par exemple une sonde submersible "Sonde CO2 Membrane/NDIR" commercialisée par AquaMS. La concentration de CO2 dissout peut également être obtenue par mesure du pH (contenu en ion hydrogène) et du kH (contenu en ion carbonate). Le gaz carbonique qui se dégage du mélange est mesuré avec le dispositif tel que précédemment décrit. La concentration du gaz carbonique dissout dans l'eau est estimée. Les paramètres du modèle sont estimés de façon à minimiser l'erreur entre la concentration de gaz carbonique estimée et les concentrations physiques des mélanges étalon.

Calibration sur des volontaires par apprentissage

Pour estimer les paramètres liés aux propriétés de la peau et du sang, et aussi pour calibrer les paramètres des modèles, on peut réaliser des mesures d'apprentissage sur des volontaires pour lesquels des mesures de référence sont réalisées. Ces mesures de référence peuvent être des dosages sur des prélèvements sanguins, ou des mesures dans l'air exhalé, ou une mesure de pression transcutanée à l'aide d'un dispositif du commerce comme un dispositif de mesure électrochimique. Les coefficients du modèle qui ne sont pas mesurés par des capteurs sont estimés en prenant une valeur moyenne qui s'appliquera sur l'ensemble des mesures d'apprentissage. Ces paramètres sont estimés en minimisant l'écart quadratique entre les pressions estimées par le modèle à partir des mesures réalisées par un dispositif 1 tel que précédemment décrit et les pressions délivrées par les dispositifs de référence.

Essai sur un utilisateur

Le dispositif 1 a été appliqué contre l'avant-bras au niveau du pli du coude d'un utilisateur sain subissant un test d'exercice cardiopulmonaire. Selon ce test, l'utilisateur est placé sur un vélo et module son effort en fonction de la vitesse de pédalage. Après une période de repos, d'une durée de 9 minutes, il réalise 4 paliers de 5 minutes en augmentant progressivement la vitesse de pédalage. Il effectue ensuite deux paliers de récupération : l'un à vitesse lente, l'autre au repos. La figure 7B montre une évolution de la pression partielle de $CO_2$ $P_{CO2}^{10}$ dans la chambre de collecte, en mettant en oeuvre les étapes 100 à 120 précédemment décrites. Les points de mesure sont représentés par des croix sombres. L'axe des ordonnées correspond à la pression partielle estimée, tandis que l'axe des abscisses correspond au temps. Compte tenu de l'importance du bruit, relativement à la dynamique du signal, les valeurs mesurées ont fait l'objet d'un lissage soit par une moyenne glissante de largeur 100 s (courbe a), soit par un polynôme d'ordre 3 (courbe b). Les valeurs lissées sont cohérentes avec l'évolution de la pression partielle de $CO_2$ dans le sang lors d'un exercice cardiopulmonaire suivant un tel protocole.

**Revendications**

1. Dispositif de mesure (1), destiné à être disposé sur la peau d'un utilisateur, l'utilisateur étant un être humain ou un animal, le dispositif s'étendant entre une face de contact (4), destinée à être appliquée contre la peau de l'utilisateur et une extrémité distale (6), le dispositif comportant une paroi latérale (5), s'étendant entre la face de contact et l'extrémité distale, le dispositif comportant :

   - au niveau de la face de contact, au moins une ouverture (11), configurée pour collecter un gaz d'intérêt transcutané, émis à travers la peau, l'ouverture étant pratiquée à travers la face de contact;
   - une chambre de collecte (10), s'étendant entre la face de contact et une chambre de mesure (20);
   - un capteur de gaz (23), disposé dans la chambre de mesure, et configuré pour mesurer une concentration de gaz d'intérêt s'écoulant dans la chambre de mesure ;
   - une unité de traitement et de calcul (18), configurée pour estimer une teneur en gaz d'intérêt dans le corps de l'utilisateur à partir de la mesure issue du capteur de gaz;
- une source de chaleur, configurée pour porter la face de contact à une température supérieure à 37°C ;

le dispositif étant **caractérisé en ce qu'**il comporte :

- une ouverture (15), formant une admission d'air, ménagée à travers la paroi latérale (5), configurée pour admettre de l'air ambiant dans la chambre de collecte (10);
de telle sorte que sous l'effet de la température produite par la source de chaleur, un courant de convection d'air s'établit, l'air admis dans la chambre de collecte (10) à travers la paroi latérale entraînant le gaz d'intérêt transcutané vers la chambre de mesure (20), l'air et le gaz d'intérêt transcutané formant un mélange gazeux s'étendant dans la chambre de mesure.

2. Dispositif selon la revendication 1, dans lequel le gaz d'intérêt est du dioxyde de carbone.

3. Dispositif selon l'une quelconque des revendications précédentes, comportant un capteur d'humidité (25) pour déterminer un niveau d'humidité dans la chambre de mesure (20), l'unité de traitement et de calcul (18) étant configurée pour prendre en compte le niveau d'humidité dans la chambre de mesure (20) pour estimer une concentration de gaz d'intérêt dans la chambre de mesure.

4. Dispositif selon l'une quelconque des revendications précédentes, comportant un capteur de pression (26), pour déterminer une pression dans la chambre de mesure (20), l'unité de traitement et de calcul (18) étant configurée pour prendre en compte la pression dans la chambre de mesure pour estimer une pression partielle de gaz d'intérêt dans la chambre de collecte (10).

5. Dispositif selon l'une quelconque des revendications précédentes, dans lequel le capteur de gaz (23) est un capteur optique, comportant une source de rayonnement infra-rouge (21) et un photodétecteur (22), la source de rayonnement infra-rouge et le photodétecteur étant agencés de telle sorte que le mélange gazeux s'écoule, dans la chambre de mesure (20), entre la source de rayonnement infra-rouge (21) et le photodétecteur (22), le photodétecteur comportant deux voies de mesures ($22_1$, $22_2$) configurées pour mesurer respectivement un rayonnement infra-rouge, émis par la source de rayonnement infra-rouge (21), et s'étant propagé à travers la chambre de mesure dans :

- une bande spectrale de mesure ($\lambda_1$), correspondant à une bande spectrale d'absorption du gaz d'intérêt ;
- une bande spectrale de référence ($\lambda_2$), considérée comme non absorbée par le gaz d'intérêt.

6. Dispositif selon l'une quelconque des revendications précédentes, comportant :

- une chambre de mesure auxiliaire (30), configurée pour être occupée par l'air ambiant s'étendant autour du dispositif (1);
- un capteur auxiliaire de gaz d'intérêt (33), disposé dans la chambre de mesure auxiliaire, et configuré pour mesurer une concentration de gaz d'intérêt dans l'air ambiant occupant la chambre de mesure auxiliaire ;
l'unité de traitement et de calcul (18) étant configurée pour prendre en compte la mesure du capteur auxiliaire pour estimer la teneur en gaz d'intérêt dans le corps de l'utilisateur.

7. Dispositif selon la revendication 6, dans lequel le capteur auxiliaire de gaz d'intérêt (33) est un capteur optique, comportant une source auxiliaire de rayonnement infra-rouge (31) et un photodétecteur auxiliaire (32), la source auxiliaire de rayonnement infra-rouge et le photodétecteur auxiliaire étant agencés de telle sorte que l'air ambiant s'étend, dans la chambre de mesure auxiliaire (30), entre la source auxiliaire de rayonnement infra-rouge et le photodétecteur auxiliaire (32), le photodétecteur auxiliaire comportant deux voies de mesures configurées pour mesurer respectivement un rayonnement infra-rouge, émis par la source auxiliaire de rayonnement infra-rouge, et s'étant propagé à travers la chambre de mesure auxiliaire dans :

- une bande spectrale de mesure ($\lambda_1$), correspondant à une bande spectrale d'absorption du gaz d'intérêt ;
- une bande spectrale de référence ($\lambda_2$), considérée comme non absorbée par le gaz d'intérêt.

8. Dispositif selon l'une quelconque des revendications précédentes, comportant :

- un capteur de pression (36) de l'air ambiant, l'air ambiant s'étendant autour du dispositif;
- et/ou un capteur de température (34) de l'air ambiant ;

- et/ou un capteur de pression (19) dans la chambre de collecte ;
- et/ou un capteur de température (17) dans la chambre de collecte ;
- et/ou un capteur de température (17'), configuré pour mesurer une température de la peau ;
- et/ou un capteur de température (24) dans la chambre de mesure.

**9.** Procédé d'estimation d'une teneur en gaz d'intérêt dans le sang d'un utilisateur, à l'aide d'un dispositif (1) selon l'une quelconque des revendications précédentes, le dispositif étant appliqué de telle sorte que la face de contact (4) est disposée en regard de la peau de l'utilisateur, le procédé comportant un échauffement de la face de contact ainsi qu'une admission d'air ambiant à travers la paroi latérale (5) du dispositif, le procédé comportant :

a) une estimation d'une concentration de gaz d'intérêt dans la chambre de mesure (20);
b) à partir de la concentration de gaz d'intérêt résultant de l'étape a), calcul d'une pression partielle de gaz d'intérêt dans la chambre de collecte (10);
c) à partir de la pression partielle de gaz d'intérêt dans la chambre de collecte (10), résultant de l'étape b), estimation d'une pression partielle de gaz d'intérêt dissout dans le sang de l'utilisateur.

**10.** Procédé selon la revendication 9, dans lequel l'étape c) comporte les sous-étapes :

(i) à partir de la pression partielle de gaz d'intérêt dans la chambre de collecte, résultant de l'étape b), estimation d'une pression partielle de gaz d'intérêt transcutané, ayant diffusé à travers la peau de l'utilisateur;
(ii) à partir de la pression partielle de gaz d'intérêt transcutané résultant de la sous-étape (i), estimation d'une pression partielle de gaz d'intérêt dissout dans le sang de l'utilisateur.

**11.** Procédé selon l'une quelconque des revendications 9 à 10, comportant également une mesure d'une concentration de gaz d'intérêt dans l'air ambiant, la concentration ainsi mesurée étant prise en compte dans l'étape c).

**12.** Procédé selon l'une quelconque des revendications 9 à 11, comportant une mesure d'une pression de l'air ambiant, la pression de l'air ambiant étant prise en compte dans l'étape c).

**13.** Procédé selon l'une quelconque des revendications 9 à 12, dans lequel l'étape a) est mise en oeuvre à l'aide d'un capteur de gaz (23), disposé dans la chambre de mesure (20), et formé par une source de rayonnement infra-rouge (21) et par un photodétecteur (22) comportant deux voies de mesures ($22_1$, $22_2$) configurées pour mesurer respectivement un rayonnement infra-rouge, émis par la source de rayonnement infra-rouge et s'étant propagé à travers la chambre de mesure (20) dans :

- une bande spectrale de mesure ($\lambda_1$), correspondant à une bande spectrale d'absorption du gaz d'intérêt ;
- une bande spectrale de référence ($\lambda_2$) considérée comme non absorbée par le gaz d'intérêt ;
et dans lequel l'étape a) comporte
- une comparaison de signaux ($U(\lambda_1)$, $U(\lambda_2)$) respectivement générés par les deux voies de mesure ;
- une résolution d'un polynôme du second degré, mettant en oeuvre la comparaison ainsi que des paramètres prédéterminés, de façon à estimer la concentration de gaz d'intérêt dans la chambre de mesure.

**14.** Procédé selon la revendication 13, dans lequel la racine du polynôme est une puissance non entière de la concentration de gaz d'intérêt dans la chambre de mesure (20).

**15.** Procédé selon l'une quelconque des revendications 9 à 14, dans lequel le gaz d'intérêt est le dioxyde de carbone.

**Patentansprüche**

**1.** Messvorrichtung (1) zur Anordnung auf der Haut eines Benutzers, wobei der Benutzer ein Mensch oder ein Tier ist, wobei sich die Vorrichtung zwischen einer Kontaktfläche (4) zur Applikation auf der Haut des Benutzers und einem distalen Ende (6) erstreckt, wobei die Vorrichtung eine Seitenwand (5) aufweist, die sich zwischen der Kontaktfläche und dem distalen Ende erstreckt, wobei die Vorrichtung Folgendes aufweist:

- an der Kontaktfläche wenigstens eine Öffnung (11), die dazu ausgebildet ist, ein transkutanes Gas von Interesse zu sammeln, das durch die Haut abgegeben wird, wobei die Öffnung durch die Kontaktfläche hindurch ausgebildet ist;

- eine Sammelkammer (10), die sich zwischen der Kontaktfläche und einer Messkammer (20) erstreckt;
- einen Gassensor (23), der in der Messkammer angeordnet ist und dazu ausgebildet ist, eine Konzentration von Gas von Interesse zu messen, das in der Messkammer strömt;
- eine Verarbeitungs- und Recheneinheit (18), die dazu ausgebildet ist, einen Gehalt an Gas von Interesse im Körper des Benutzers anhand der Messung aus dem Gassensor zu schätzen;
- eine Wärmequelle, die dazu ausgebildet ist, die Kontaktfläche auf eine Temperatur höher als 37 °C zu bringen;

wobei die Vorrichtung **dadurch gekennzeichnet ist, dass** sie Folgendes aufweist:

- eine Öffnung (15), die einen Lufteinlass bildet, die durch die Seitenwand (5) hindurch vorgesehen ist, die dazu ausgebildet ist, Umgebungsluft in die Sammelkammer (10) einzulassen;

so dass sich unter der Einwirkung der Temperatur, die von der Wärmequelle erzeugt wird, eine Luftkonvektionsströmung einstellt, wobei die Luft, die durch die Seitenwand in die Sammelkammer (10) eingelassen wird, das transkutane Gas von Interesse zur Messkammer (20) mitnimmt, wobei die Luft und das transkutane Gas von Interesse ein Gasgemisch bilden, das sich in der Messkammer erstreckt.

2. Vorrichtung nach Anspruch 1, wobei das Gas von Interesse Kohlendioxid ist.

3. Vorrichtung nach einem der vorhergehenden Ansprüche, aufweisend einen Feuchtesensor (25) zur Bestimmung einer Feuchtigkeit in der Messkammer (20), wobei die Verarbeitungs- und Recheneinheit (18) dazu ausgebildet ist, die Feuchtigkeit in der Messkammer (20) zu berücksichtigen, um eine Konzentration von Gas von Interesse in der Messkammer zu schätzen.

4. Vorrichtung nach einem der vorhergehenden Ansprüche, aufweisend einen Drucksensor (26) zur Bestimmung eines Drucks in der Messkammer (20), wobei die Verarbeitungs- und Recheneinheit (18) dazu ausgebildet ist, den Druck in der Messkammer zu berücksichtigen, um einen Partialdruck von Gas von Interesse in der Sammelkammer (10) zu schätzen.

5. Vorrichtung nach einem der vorhergehenden Ansprüche, wobei der Gassensor (23) ein optischer Sensor ist, der eine Infrarotstrahlungsquelle (21) und einen Photodetektor (22) aufweist, wobei die Infrarotstrahlungsquelle und der Photodetektor so angeordnet sind, dass das Gasgemisch in der Messkammer (20) zwischen der Infrarotstrahlungsquelle (21) und dem Photodetektor (22) strömt, wobei der Photodetektor zwei Messkanäle (221, 222) aufweist, die dazu ausgebildet sind, jeweils eine Infrarotstrahlung zu messen, die von der Infrarotstrahlungsquelle (21) abgegeben wird und sich durch die Messkammer ausgebreitet hat, in:

- einem Messspektralband ($\lambda_1$), das einem Absorptionsspektralband des Gases von Interesse entspricht;
- einem Referenzspektralband ($\lambda_2$), das als nicht durch das Gas von Interesse absorbiert erachtet wird.

6. Vorrichtung nach einem der vorhergehenden Ansprüche, aufweisend:

- eine Hilfsmesskammer (30), die dazu ausgebildet ist, von der Umgebungsluft eingenommen zu sein, die sich um die Vorrichtung (1) herum erstreckt;
- einen Hilfssensor für Gas von Interesse (33), der in der Hilfsmesskammer angeordnet ist und dazu ausgebildet ist, eine Konzentration von Gas von Interesse in der Umgebungsluft zu messen, die die Hilfsmesskammer einnimmt;

wobei die Verarbeitungs- und Recheneinheit (18) dazu ausgebildet ist, die Messung des Hilfssensors zu berücksichtigen, um den Gehalt an Gas von Interesse im Körper des Benutzers zu schätzen.

7. Vorrichtung nach Anspruch 6, wobei der Hilfssensor für Gas von Interesse (33) ein optischer Sensor ist, der eine Hilfs-Infrarotstrahlungsquelle (31) und einen Hilfsphotodetektor (32) aufweist, wobei die Hilfs-Infrarotstrahlungsquelle und der Hilfsphotodetektor so angeordnet sind, dass sich die Umgebungsluft in der Hilfsmesskammer (30) zwischen der Hilfs-Infrarotstrahlungsquelle und dem Hilfsphotodetektor (32) erstreckt, wobei der Hilfsphotodetektor zwei Messkanäle aufweist, die dazu ausgebildet sind, jeweils eine Infrarotstrahlung zu messen, die von der Hilfs-Infrarotstrahlungsquelle abgegeben wird und sich durch die Hilfsmesskammer ausgebreitet hat, in:

- einem Messspektralband ($\lambda_1$), das einem Absorptionsspektralband des Gases von Interesse entspricht;

- einem Referenzspektralband ($\lambda_2$), das als nicht durch das Gas von Interesse absorbiert erachtet wird.

8. Vorrichtung nach einem der vorhergehenden Ansprüche, aufweisend:

- einen Sensor für den Druck (36) der Umgebungsluft, wobei sich die Umgebungsluft um die Vorrichtung herum erstreckt;
- und/oder einen Sensor für die Temperatur (34) der Umgebungsluft;
- und/oder einen Drucksensor (19) in der Sammelkammer;
- und/oder einen Temperatursensor (17) in der Sammelkammer;
- und/oder einen Temperatursensor (17'), der dazu ausgebildet ist, eine Temperatur der Haut zu messen;
- und/oder einen Temperatursensor (24) in der Messkammer.

9. Verfahren zur Schätzung eines Gehalts an Gas von Interesse im Blut eines Benutzers mithilfe einer Vorrichtung (1) nach einem der vorhergehenden Ansprüche, wobei die Vorrichtung so appliziert wird, dass die Kontaktfläche (4) an der Haut des Benutzers angeordnet ist, wobei das Verfahren ein Erwärmen der Kontaktfläche sowie ein Einlassen von Umgebungsluft durch die Seitenwand (5) der Vorrichtung aufweist, wobei das Verfahren Folgendes aufweist:

a) ein Schätzen einer Konzentration von Gas von Interesse in der Messkammer (20);
b) anhand der Konzentration von Gas von Interesse, die aus Schritt a) hervorgeht, Berechnen eines Partialdrucks von Gas von Interesse in der Sammelkammer (10);
c) anhand des Partialdrucks von Gas von Interesse in der Sammelkammer (10), der aus Schritt b) hervorgeht, Schätzen eines Partialdrucks von Gas von Interesse, das im Blut des Benutzers gelöst ist.

10. Verfahren nach Anspruch 9, wobei der Schritt c) die folgenden Unterschritte aufweist:

(i) anhand des Partialdrucks von Gas von Interesse in der Sammelkammer, der aus Schritt b) hervorgeht, Schätzen eines Partialdrucks von transkutanem Gas von Interesse, das durch die Haut des Benutzers hindurch diffundiert ist;
(ii) anhand des Partialdrucks von transkutanem Gas von Interesse, der aus Schritt (i) hervorgeht, Schätzen eines Partialdrucks von Gas von Interesse, das im Blut des Benutzers gelöst ist.

11. Verfahren nach einem der Ansprüche 9 bis 10, ebenfalls aufweisend ein Messen einer Konzentration von Gas von Interesse in der Umgebungsluft, wobei die so gemessene Konzentration im Schritt c) berücksichtigt wird.

12. Verfahren nach einem der Ansprüche 9 bis 11, aufweisend ein Messen eines Drucks der Umgebungsluft, wobei der Druck der Umgebungsluft im Schritt c) berücksichtigt wird.

13. Verfahren nach einem der Ansprüche 9 bis 12, wobei der Schritt a) mithilfe eines Gassensors (23) durchgeführt wird, der in der Messkammer (20) angeordnet ist und von einer Infrarotstrahlungsquelle (21) und von einem Photodetektor (22) gebildet ist, der zwei Messkanäle (221, 222) aufweist, die dazu ausgebildet sind, jeweils eine Infrarotstrahlung zu messen, die von der Infrarotstrahlungsquelle abgegeben wird und sich durch die Messkammer (20) ausgebreitet hat, in:

- einem Messspektralband ($\lambda_1$), das einem Absorptionsspektralband des Gases von Interesse entspricht;
- einem Referenzspektralband ($\lambda_2$), das als nicht durch das Gas von Interesse absorbiert erachtet wird;
und wobei der Schritt a) Folgendes aufweist
- ein Vergleichen von Signalen ($U(\lambda_1)$, $U(\lambda_2)$), die jeweils von den zwei Messkanälen erzeugt werden;
- ein Lösen eines Polynoms zweiten Grades unter Verwendung des Vergleichs sowie vorbestimmter Parameter, um die Konzentration von Gas von Interesse in der Messkammer zu schätzen.

14. Verfahren nach Anspruch 13, wobei die Wurzel des Polynoms eine nichtganzzahlige Potenz der Konzentration von Gas von Interesse in der Messkammer (20) ist.

15. Verfahren nach einem der Ansprüche 9 bis 14, wobei das Gas von Interesse das Kohlendioxid ist.

**Claims**

1. Measuring device (1), intended to be placed on the skin of a user, the user being a human being or an animal, the device extending between a contact face (4), intended to be applied against the skin of the user, and a far end (6), the device comprising a side wall (5) that extends between the contact face and the far end, the device comprising:

   - on the contact face, at least one aperture (11) that is configured to collect a transcutaneous gas of interest emitted through the skin, the aperture being produced through the contact face;
   - a collecting chamber (10) that extends between the contact face and a measuring chamber (20);
   - a gas sensor (23), placed in the measuring chamber, and configured to measure a concentration of the gas of interest flowing through the measuring chamber;
   - a processing and computing unit (18), configured to estimate a gas-of-interest content in the body of the user on the basis of the measurement taken by the gas sensor;
   - a heat source, configured to bring the contact face to a temperature above 37°C;

   the device being **characterized in that** it comprises:

   - an aperture (15) that forms an air intake, said aperture being produced through the side wall (5) and configured to admit ambient air into the collecting chamber (10);
   - such that, under the effect of the temperature produced by the heat source, a convection current of air is generated, the air admitted into the collecting chamber (10) through the side wall entraining the transcutaneous gas of interest to the measuring chamber (20), the air and the transcutaneous gas of interest forming a gas mixture extending into the measuring chamber.

2. Device according to Claim 1, wherein the gas of interest is carbon dioxide.

3. Device according to either one of the preceding claims, comprising a humidity sensor (25) for determining a humidity level in the measuring chamber (20), the processing and computing unit (18) being configured to take into account the humidity level in the measuring chamber (20) to estimate a concentration of gas of interest in the measuring chamber.

4. Device according to any one of the preceding claims, comprising a pressure sensor (26) for determining a pressure in the measuring chamber (20), the processing and computing unit (18) being configured to take into account the pressure in the measuring chamber to estimate a partial pressure of gas of interest in the collecting chamber (10).

5. Device according to any one of the preceding claims, wherein the gas sensor (23) is an optical sensor that comprises an infrared radiation source (21) and a photodetector (22), the infrared radiation source and the photodetector being arranged such that the gas mixture flows, in the measuring chamber (20), between the infrared radiation source (21) and the photodetector (22), the photodetector comprising two measurement channels ($22_1$, $22_2$) configured to respectively measure infrared radiation, emitted by the infrared radiation source (21), and having propagated through the measuring chamber in:

   - a measurement spectral band ($\lambda_1$) that corresponds to an absorption spectral band of the gas of interest;
   - a reference spectral band ($\lambda_2$), considered as not absorbed by the gas of interest.

6. Device according to any one of the preceding claims, comprising:

   - an auxiliary measuring chamber (30), configured to be occupied by the ambient air extending around the device (1);
   - an auxiliary gas-of-interest sensor (33), placed in the auxiliary measuring chamber, and configured to measure a concentration of gas of interest in the ambient air occupying the auxiliary measuring chamber;
   the processing and computing unit (18) being configured to take into account the measurement of the auxiliary sensor to estimate the gas-of-interest content in the body of the user.

7. Device according to Claim 6, wherein the auxiliary gas-of-interest sensor (33) is an optical sensor that comprises an auxiliary infrared radiation source (31) and an auxiliary photodetector (32), the auxiliary infrared radiation source and the auxiliary photodetector being arranged such that ambient air extends, in the auxiliary measuring chamber (30), between the auxiliary infrared radiation source and the auxiliary photodetector (32), the auxiliary photodetector comprising two measurement channels configured to respectively measure infrared radiation, emitted by the auxiliary infrared radiation source, and having propagated through the auxiliary measuring chamber in:

- a measurement spectral band ($\lambda_1$) that corresponds to an absorption spectral band of the gas of interest;
- a reference spectral band ($\lambda_2$), considered as not absorbed by the gas of interest.

8. Device according to any one of the preceding claims, comprising:

- a sensor (36) of ambient air pressure, the ambient air extending around the device;
- and/or a sensor (34) of ambient air temperature;
- and/or a sensor (19) of pressure in the collecting chamber;
- and/or a sensor (17) of temperature in the collecting chamber;
- and/or a temperature sensor (17'), configured to measure a skin temperature;
- and/or a sensor (24) of temperature in the measuring chamber.

9. Method for estimating a gas-of-interest content in the blood of a user, using a device (1) according to any one of the preceding claims, the device being applied such that the contact face (4) is placed facing the skin of the user, the method comprising heating the contact face and admitting ambient air through the side wall (5) of the device, the method comprising:

a) an estimation of a concentration of gas of interest in the measuring chamber (20);
b) on the basis of the concentration of gas of interest resulting from step a), computing a partial pressure of gas of interest in the collecting chamber (10);
c) on the basis of the partial pressure of gas of interest in the collecting chamber (10), resulting from step b), estimating a partial pressure of gas of interest dissolved in the blood of the user.

10. Method according to Claim 9, wherein step c) comprises the sub-steps:

(i) on the basis of the partial pressure of gas of interest in the collecting chamber, resulting from step b), estimating a partial pressure of transcutaneous gas of interest having diffused through the skin of the user;
(ii) on the basis of the partial pressure of transcutaneous gas of interest resulting from sub-step (i), estimating a partial pressure of gas of interest dissolved in the blood of the user.

11. Method according to either one of Claims 9 and 10, also comprising measuring a concentration of gas of interest in the ambient air, the concentration thus measured being taken into account in step c).

12. Method according to any one of Claims 9 to 11, comprising measuring an ambient air pressure, the ambient air pressure being taken into account in step c).

13. Method according to any one of Claims 9 to 12, wherein step a) is implemented using a gas sensor (23), placed in the measuring chamber (20), and formed by an infrared radiation source (21) and by a photodetector (22) comprising two measurement channels ($22_1$, $22_2$) configured to respectively measure infrared radiation, emitted by the infrared radiation source, and having propagated through the measuring chamber (20) in:

- a measurement spectral band ($\lambda_1$) that corresponds to an absorption spectral band of the gas of interest;
- a reference spectral band ($\lambda_2$), considered as not absorbed by the gas of interest;
and wherein step a) comprises
- a comparison of signals ($U(\lambda_1)$, $U(\lambda_2)$) respectively generated by the two measurement channels;
- solving a second-degree polynomial, this implementing the comparison and predetermined parameters, so as to estimate the concentration of gas of interest in the measuring chamber.

14. Method according to Claim 13, wherein the root of the polynomial is a non-integer power of the concentration of gas of interest in the measuring chamber (20).

15. Method according to any one of Claims 9 to 14, wherein the gas of interest is carbon dioxide.

**Fig. 1A**

**Fig. 1B**

**Fig. 2A**

**Fig. 2B**

**Fig. 3A**

**Fig. 3B**

**Fig. 3C**

**Fig. 4**

$\{m_1, n_1, m_2, n_2, q, u_1, u_2\}$

90

100

$C_{CO2}$

110

$P_{CO2}^{20}$

120

$P_{CO2}^{10}$

130

$P_{CO2}^{tc}$

140

$P_{CO2}^{blood}$

**Fig. 5**

**Fig. 6A**

**Fig. 6B**

**Fig. 7A**

**Fig. 7B**

**EP 3 979 911 B1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2016151009 A1
- US 2013281806 A1
- US 2010130842 A1
- US 3987303 A
- US 2001034479 A1
- FR 2408353 A1

**Littérature non-brevet citée dans la description**

- **ELETR S.** Cutaneous monitoring of systemic PCO2 on patients in the respiratory intensive care unit being weaned from the ventilator. *Anaesthesiol Scand,* 1978, vol. 22 (68), 123-127
- **J.W. SEVERINGHAUS.** The current status of transcutaneous blood gas analysis and monitoring. *Blood Gas News,* 1998, vol. 7 (2), 4-9